# EUROPEAN PATENT APPLICATION

(11) **EP 4 371 993 A1**
(43) Date of publication of application: **22.05.2024**
(21) Application number: 22841427.2
(22) Date of filing: 13.07.2022
(51) Int. Cl.: C07F 9/6553, A61K 31/67, A61P 29/00, A61P 19/02, A61P 25/00, A61P 37/02

(54) **ASYMMETRIC GPR84 ANTAGONIST AND USE THEREOF**

(30) Priority: 15.07.2021 CN 202110801592
(71) Applicant: Shanghai Institute of Materia Medica, Chinese Academy of Sciences, Pudong, Shanghai 201203 (CN)
(72) Inventor: NAN, Fajun, Shanghai 201203 (CN); XIE, Xin, Shanghai 201203 (CN); LI, Shaoxian, Shanghai 201203 (CN); ZHANG, Qing, Shanghai 201203 (CN); CHEN, Linhai, Shanghai 201203 (CN); FANG, Youchen, Shanghai 201203 (CN)
(74) Representative: Mewburn Ellis LLP
(86) International application number: PCT/CN2022/105507
(87) International publication number: WO 2023/284794

(57) **Abstract**

An asymmetric GPR84 antagonist and a use thereof, the GPR84 antagonist having a structure as shown in formula I. The compound represented by formula I has GPR84 antagonistic activity, can competitively inhibit GPR84 agonist-induced receptor activation, and can be used to treat various diseases associated with the high expression or hyperexcitability of GPR84, such as multiple sclerosis, inflammatory bowel disease, organ fibrosis, arthritis, etc.

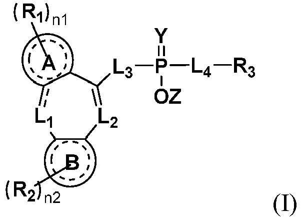

## Description

### Technical field

The present invention relates to a ligand molecule of G protein-coupled receptor 84 (GPR84). The ligand molecule of the present invention has an antagonistic activity of GPR84, can competitively inhibit the activation of the receptor by an agonist of GPR84, and can be used to treat various diseases related to high expression or hyperexcitability of GPR84, such as multiple sclerosis, inflammatory bowel disease, organ fibrosis, arthritis and the like.

### Background technique

GPR84 is a G protein-coupled receptor discovered in 2001. It is coupled to the Gi pathway and can be activated by medium-chain fatty acids (MCFAs) with a chain length of C9-C14. After activation, GPR84 can inhibit adenylyl cyclase and reduce the production of cAMP. GPR84 is expressed in a variety of tissues or organs, such as heart, lung, kidney, liver, bone marrow, fat, etc., especially in myeloid cells related to the innate immune system, including monocytes, macrophages and neutrophils in the peripheral blood, and microglia in the central nervous system. Under physiological conditions, the expression of GPR84 in leukocytes and adipocytes is low, but acute inflammatory stimuli (such as lipopolysaccharide LPS, TNFα, or inflammatory responses associated with many diseases) can induce a significant upregulation of GPR84 expression. The activation of GPR84 can enhance the phagocytosis of macrophages, promote the chemotaxis of immune cells, increase the secretion of pro-inflammatory cytokines (IL-12 p40), thereby amplifying the body's inflammatory response; in contrast the knockout of GPR84 resultes in decreased secretion of pro-inflammatory cytokines (IL-1, IL-6, and TNF) in macrophages, along with increased secretion of Th2 cytokines (IL-4, IL-5, and IL-13) in T cells. The above studies have confirmed the pro-inflammatory role of GPR84 in the body's metabolic regulation and immune response, and subsequent studies have revealed that GPR84 is related to the development of various inflammatory and metabolic diseases, such as multiple sclerosis (MS, Glia 2007, 55, 790 -800), inflammatory bowel disease (IBD, J. Med. Chem. 2020, 63, 13526-13545), organ fibrosis (J. Clin. Med. 2020, 9, 4, Am. J. Pathol.2018, 188, 1132-1148), arthritis (Curr. Opin. Clin. Nutr. Metab. Care 2011, 14, 322-327) and the like. Therefore, GPR84 is a potential target for the treatment of the above diseases, and GPR84 antagonists are expected to be used in the treatment of these diseases.

To date, only the patents of Galapagos (Belgium) and Liminal (Canada) report GPR84 antagonists. The compounds from Galapagos have tetrahydroisoquinolinopyrimidinone (or pyridone) as a core (WO2013092791, WO2014095798, WO2015197550, WO2016169911). The representative compound GLPG1205, a GPR84 negative allosteric agent with high activity (J. Med. Chem. 2020, 63, 13526-13545), was previously entered into phase II clinical study as a candidate drug for the treatment of inflammatory bowel disease, but the trial was terminated due to a lack of significant difference in efficacy from the placebo group. The compound is undergoing a second phase II clinical evaluation for the treatment of idiopathic pulmonary fibrosis. PBI-4050 and PBI-4547 from Liminal are non-selective GPR84 antagonists, and their structures are 3-n-pentyl phenylacetate sodium and 3,5-di-n-pentyl phenylacetate sodium, both of which are analogues of fatty acids. The antagonistic activities on GPR84 are at the micromolar level, and they have agonistic activity on fatty acid receptors GPR40 and GPR120. The phase II clinical trial of PBI-4050 for the treatment of idiopathic pulmonary fibrosis has been completed, and the phase II/III clinical trial for the treatment of Alström is currently underway; and PBI-4547 is undergoing phase I clinical study as a drug candidate for nonalcoholic steatohepatitis.

Nan fajun et al. have reported a class of GPR84 antagonists with a phosphodiester structure having high activity (WO2018161831), in which both ester groups are tricyclic structures. The representative compound XYF573c can significantly alleviate the symptoms of DSS-induced enteritis in mice and the effect is better than that of sulfasalazine at the same dose. In addition, tissue distribution studies show that XYF573c can be selectively distributed in the intestinal tract (AUC₀₋₈ₕ=24447.42 h*ng/mL, oral administration: 5mg/kg), while the exposure in peripheral blood is relatively low (AUC₀₋₈ₕ=666.53h *ng/mL, oral administration: 5mg/kg), so XYF573c mainly acts on the up-regulated GPR84 in intestinal immune cells and inhibits the inflammatory response mediated by GPR84 to treat enteritis, and its distribution in peripheral blood shows that it can also work in part by inhibiting the migration of peripheral immune cells. The targeted intestinal distribution of such GPR84 antagonists suggests their suitability for the treatment of inflammatory bowel disease, but their poor oral absorption limits their application in the treatment of diseases such as organ fibrosis, multiple sclerosis, arthritis and the like.

The oral absorption of compounds is closely related to the lipid-water partition coefficients (e.g., CLogP, ALogP), and the CLogP of compounds with better drug-like properties needs to be less than 5 (Lipinski's Rule-of-Five), while the CLogP of XYF573c is 6.419, which is still a large space for optimization. In the phosphodiester structure of the original GPR84 antagonist, both ester groups are strongly lipophilic tricyclic structures, and this symmetric structural feature not only limits the expansion of the chemical space of the compounds, but also greatly hampers the optimization of the drug-like properties of the compounds (e.g., CLogP, ALogP, etc.).

### Summary of the invention

The object of the present invention is to provide a novel GPR84 antagonist, a preparation process and use thereof.

The first aspect of the invention provides a compound represented by general formula (I), or an enantiomer, diastereoisomer, racemate, or a pharmaceutically acceptable salt thereof, 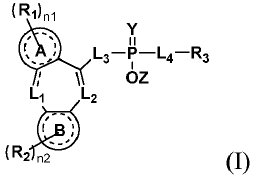
wherein Y is O or S;
Z is H, or an ion of the following metals: Li, Na, K, Ca, Mg, Cu, Fe, Zn, Al, Mn, or a conjugate acid of the following bases: NH₃, arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxycobalamin, isopropylamine, lysine, methyl glucosamine, morpholine, piperazine, piperidine, polyamine resin, procaine, purine, theobromine, triethylamine, trimethylamine, tripropylamine, trometamol, imidazole;
L₁ is none, O, S, -CH=CH-, -CO-, -C(=CH₂)-, substituted or unsubstituted C₁-C₆ alkylene, -NH-, -N(C₁-C₄ alkyl)-, C₃-C₆ cycloalkyl or C₃-C₆ heterocycloalkyl, and the substitution means that there is one or more substituents selected from the group consisting of C₁-C₆ alkyl, C₁-C₆ alkoxy, halogen, and hydroxyl;
L₂ is none, CH;
ring A and ring B are each independently a C₆-C₁₀ aromatic ring, a C₃-C₁₀ cycloalkane ring, a C₃-C₁₀ heterocycloalkane ring, or a C₃-C₁₀ heteroaryl ring;
n1 and n2 are each independently 0, 1, 2, 3 or 4; R₁ and R₂ are each independently -OH, -SH, -NH₂, F, Cl, Br, I, substituted or unsubstituted -CᵣH₂ᵣ-L₇-CₛH₂ₛ₊₁, -CᵣH₂ᵣ-N(CₜH₂ₜ₊₁)-CₛH₂ₛ₊₁, substituted or unsubstituted C₁-C₆ alkyl, the above substitution means there is one or more substituents selected from the group consisting of halogen, hydroxyl, amino, -COOC₁-C₆ alkyl, -COOH; each L₇ is independently O, S, NH; each r is independently 0, 1, 2, 3, 4, 5 or 6; each s is independently 0, 1, 2, 3, 4, 5 or 6; and each t is independently 1, 2, 3, 4, 5, or 6;
L₃ and L₄ are each independently O, none, -O(C₁-C₁₀ alkylene)-, -O(C₁-C₁₀ alkylene)NH-, -O(C₁-C₁₀ alkylene)O-, -CONH-, -OCO-, -NH-, -NHCOO-, -N(C₁-C₆ alkyl)-, or C₁-C₁₀ alkylene;
R₃ is H, OH, NH₂, SH, -COOH, substituted or unsubstituted C₁-C₁₀ alkyl, substituted or unsubstituted C₂-C₁₀ alkenyl, substituted or unsubstituted C₂-C₁₀ alkynyl, substituted or unsubstituted 3-12 membered cycloalkane ring, substituted or unsubstituted C₆-C₁₄ aromatic ring, substituted or unsubstituted amino, substituted or unsubstituted 4-10 membered heterocycloalkane ring, substituted or unsubstituted 4-10 membered heteroaromatic ring, cholic acid, lithocholic acid, deoxycholic acid, isolithocholic acid, isodeoxycholic acid, chenodeoxycholic acid, ursodeoxycholic acid, α-muricholic acid, β-muricholic acid, γ-muricholic acid, ω-muricholic acid; and the above substitution means that there is 1, 2, 3, 4, 5 or 6 substituents selected from the following group consisting of amino, C₁-C₁₀ alkyl, halogen, C₆-C₁₀ aromatic ring, C₁-C₆ alkoxy, 3-12 membered cycloalkane ring, -O-CₚH₂ₚ-O-C_{q}H_{2q+1}, nitro, oxo (=O), hydroxyl, carboxyl, -C(O)OC₁ -C₆ alkyl, -O-C₆-C₁₀ aromatic ring, -NH-(4-10 membered heteroaromatic ring), -NH-(4-10 membered heteroaromatic ring)-CONH₂; wherein each p is independently 1, 2, 3, 4, 5 or 6, and each q is independently 1, 2, 3, 4, 5 or 6;
each - - - independently represents a single or double bond.

In another preferred embodiment, ring A and ring B are each independently a benzene ring, a C₃-C₆ cycloalkane ring, a C₃-C₆ heterocycloalkane ring, or a C₃-C₆ heteroaromatic ring.

In another preferred embodiment, ring A and ring B are each independently a benzene ring, a thiophene ring, a pyrrole ring, a furan ring, a cyclohexane ring, a cyclopentane ring or a cycloheptane ring.

In another preferred embodiment, L₁ is independently none, CH₂, O, S, -CO-, -NH-; and L2 is independently none, CH.

In another preferred embodiment, n1 is 0, 1 or 2; and R₁ is F, Cl, Br, I, methoxy, ethoxy, n-propoxy, isopropoxy, trifluoromethoxy.

In another preferred embodiment, n2 is 0, 1 or 2; and R₂ is F, Cl, Br, I, methoxy, ethoxy, n-propoxy, isopropoxy, trifluoromethoxy.

In another preferred embodiment, L₃ is O or S.

In another preferred embodiment, L₄ is O, none, -NH-, -OCH₂NH-, -OCH₂CH₂NH-, -OCH₂CH₂CH₂NH-, -N(C₁-C₄ alkyl)-, -CH₂- or -CH₂CH₂-.

In the present invention, there is no particular restriction on the connection manner of the group, such as -O(C₁-C₁₀ alkylene)-, -O(C₁-C₁₀ alkylene)NH-, -CONH-, -OCO-, -NHCOO-, -OCH₂NH-, -OCH₂CH₂NH- , -OCH₂CH₂CH₂NH- and the like, which is connected from left to right, or from right to left to a connected atom or group, such as C, P, R₃. For example, for P-L4-R3, when L4 is -CONH-, it means P-CONH-R3 or R3-CONH-P. In another preferred embodiment, when L4 is defined as the above mentioned moiety, the above mentioned moiety is connected to P at the left end, and connected to R₃ at the right end.

In another preferred embodiment, R₃ is a substituted or unsubstituted C₁-C₁₀ alkyl, substituted or unsubstituted C₂-C₁₀ alkenyl, substituted or unsubstituted C₂-C₁₀ alkynyl, substituted or unsubstituted 3-12 membered cycloalkane ring, substituted or unsubstituted C₆-C₁₄ aromatic ring, substituted or unsubstituted amino, substituted or unsubstituted 4-10 membered heterocycloalkane ring, substituted or unsubstituted 4-10 membered heteroaromatic ring, cholic acid, lithocholic acid, deoxycholic acid, isodeoxycholic acid, isodeoxycholic acid, chenodeoxycholic acid, ursodeoxycholic acid, α-muricholic acid, β-muricholic acid, γ-muricholic acid, ω-muricholic acid; and the above substitution means that there is 1, 2, 3, 4, or 5 substituents selected from the following group consisting of amino, C₁-C₁₀ alkyl, halogen, C₆-C₁₀ aromatic ring, C₁- C₆ alkoxy, 3-12 membered cycloalkane ring, -O-CₚH₂ₚ-O-C_{q}H_{2q+1}, nitro, oxo (=O), hydroxyl, carboxyl, -C(O)OC₁-C₆ alkyl, -O-benzene ring; wherein each p is independently 1, 2, or 3, and each q is independently 1, 2, or 3.

In another preferred embodiment, the pharmaceutically acceptable salt is a salt obtained by reacting a compound of formula I with an inorganic base, and is selected from the group consisting of lithium salt, potassium salt, sodium salt, calcium salt, magnesium salt, copper salt, iron salt, ferrous salt, zinc salt, aluminum salt, ammonium salt, manganese salt, manganite salt; or
the pharmaceutically acceptable salt is a salt obtained by reacting a compound of formula I with an organic base, and the organic base is selected from the group consisting of NH₃, arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxycobalamin, isopropylamine, lysine, methyl glucosamine, morpholine, piperazine, piperidine, polyamine resin, procaine, purine, theobromine, triethylamine, trimethylamine, tripropylamine, trometamol, imidazole.

In another preferred embodiment, the compound is any compound prepared in the examples.

The second aspect of the present invention provides a method for preparing the compound according to the first aspect, comprising the following step: 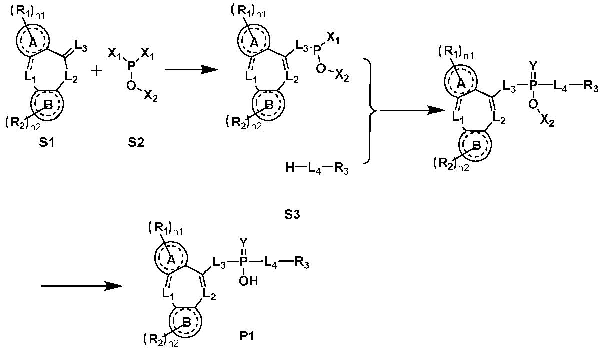
taking a compound of formula S1, a compound of formula S2 and a compound of formula S3 as raw materials and reacting to obtain the compound having the structure shown in formula P1,
wherein R₁, R₂, R₃, L₁, L₂, L₄, L₅, Y, n1, n2, ring A, and ring B are as defined above;
L₃ is O;
each X₁ is independently a dimethylamino, ethylamino, diisopropylamino;
X₂ is a cyanoethyl, allyl, tert-butyl, benzyl.

The third aspect of the present invention provides a method for preparing the compound according to the first aspect, comprising the following step: 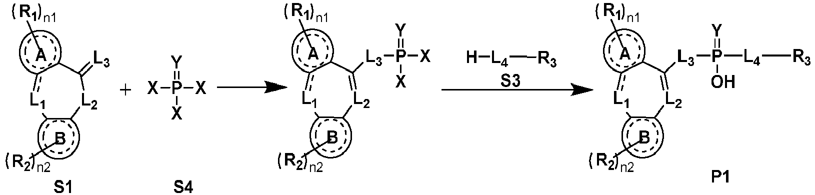
taking a compound of formula S1, a compound of formula S3 and a compound of formula S4 as raw materials and reacting to obtain the compound having the structure shown in formula P1, wherein R₁, R₂, R₃, L₁, L₂, L₄, L₅, Y, n1, n2, ring A, and ring B are as defined above;
L₃ is O or CH₂; and each X is independently F, Cl, Br or I.

The fourth aspect of the present invention provides a method for preparing the compound according to the first aspect, comprising the following step: 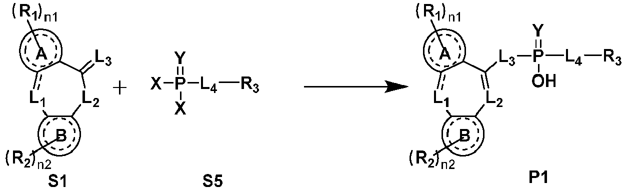
taking a compound of formula S 1 and a compound of formula S5 as raw materials and reacting to obtain the compound having the structure shown in formula P 1,
wherein R₁, R₂, R₃, L₁, L₂, L₄, L₅, Y, n1, n2, ring A, and ring B are as defined above;
L₃ is O or CH₂;
each X is independently F, Cl, Br or I.

The fifth aspect of the present invention provides a pharmaceutical composition comprising:
the compound represented by the general formula (I) according to the first aspect, or the enantiomer, diastereoisomer, racemate or pharmaceutically acceptable salt thereof; and
a pharmaceutically acceptable carrier.

The sixth aspect of the present invention provides a use of the compound according to the first aspect, or the enantiomer, diastereoisomer, racemate or pharmaceutically acceptable salt thereof or the pharmaceutical composition according to the fifth aspect:
(i) for the preparation of a GPR84 antagonist;
(ii) as a GPR84 antagonist;
(iii) for the preparation of a medicament for the treatment of a related disease caused by hyper-excitability or high expression of GPR84 receptor.

In another preferred embodiment, the disease is multiple sclerosis, inflammatory bowel disease, fibrosis, neurodegenerative disease or arthritis.

The seventh aspect of the present invention provides a method for treating a related disease caused by hyper-excitability or high expression of GPR84 receptor, comprising administering the compound of the present invention or a pharmaceutically acceptable salt to a patient in need thereof.

Unless otherwise defined, all professional and scientific terms used herein have the same meaning known as those skilled in the art. Moreover, any methods and materials similar or equivalent to those described herein may be employed in the methods of the invention. The preferred embodiments and materials described herein are for illustrative purposes only.

It should be understood that within the scope of the present invention, each of the above technical features of the present invention and each of technical features specifically described below (as in the examples) can be combined with each other to constitute a new or preferred technical solution. Due to space limitations, they will not be repeated herein.

### Description of drawings

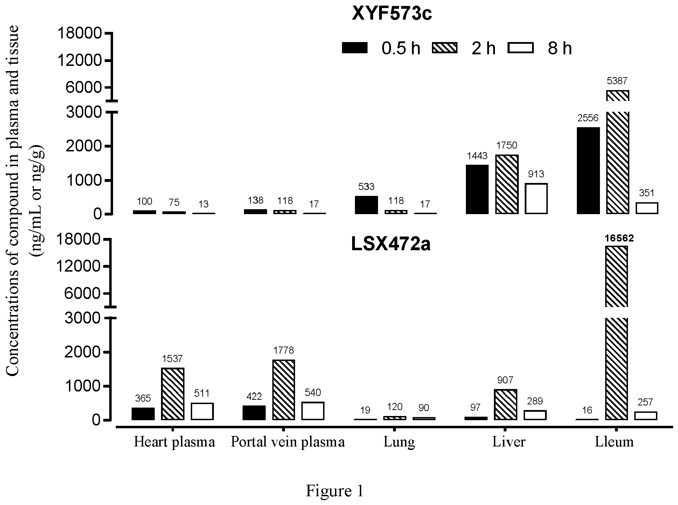

Figure 1 shows the tissue distribution of compounds XYF573c and LSX472a in ICR mice.

### Detailed description of the invention

Based on extensive and intensive studies, the inventors of the present application have developed a novel GPR84 antagonist, wherein one ester group of the phosphodiester is a tricyclic segment, and the other ester group is derived into various structural types, such as (cyclo)alkyl or substituted (cyclo)alkyl, aryl or substituted aryl, cholic acid derivatives, etc. The compound of the present application can competitively inhibit the activation of the receptor caused by the agonist of GPR84, and can be used for the preparation of a medicament for treating a related disease caused by high expression or hyperexcitability of the GPR84 receptor, and the diseases include multiple sclerosis, inflammatory bowel disease, fibrosis, neurodegenerative disease, arthritis and the like. In addition, the modification of such asymmetric structure breaks through the structural characteristics of the original symmetrical phosphodiester GPR84 antagonists, and reduces the CLogP of the compound on the basis of maintaining the GPR84 antagonistic activity, so as to provide them with better oral absorption and organ distribution properties, and facilitate the development of drugs for the treatment of organ fibrosis, arthritis, multiple sclerosis, and inflammatory bowel disease, and the like. On this basis, the present invention was accomplished. The distribution characteristics are conducive to the development of drugs for the treatment of organ fibrosis, arthritis, multiple sclerosis, and inflammatory bowel disease. On the basis of this, the present invention has been completed.

### Term

In the present invention, C₆-C₁₀ means there are 6 to 10 carbon atoms, and C₃-C₆ means there are 3 to 6 carbon atoms, and so on.

In the present invention, terms such as aromatic ring, cycloalkane ring, alkyl, alkenyl, and alkynyl have the same meanings as those familiar to those skilled in the art, unless otherwise specified. For example, alkyl refers to a saturated linear or branched hydrocarbyl; for example, -CH₃ or -CH(CH₃)₂; alkylidene refers to a remaining moiety formed by formally removing two one-valence hydrogens from a saturated hydrocarbyl, including but not limited to methene (-CH₂-), ethylene (-CH₂CH₂-), and the like. Alkoxy means -O-(alkyl), including but not limited to -OCH₃, -OCH₂CH₃ and the like. Cycloalkane ring, cycloalkyl refer to a saturated cyclic hydrocarbyl such as a cyclohexyl. Heterocycloalkyl, heterocycloalkane ring refer to a saturated cyclic hydrocarbyl containing at least one (e.g., 1, 2, 3 or 4) hetero atom (selected from N, O or S). Heteroaromatic ring, heteroaryl refers to an aromatic ring containing at least one (such as 1, 2, 3 or 4) hetero atom (selected from N, O or S).

Unless otherwise stated, the aromatic ring, heteroaryl ring, cycloalkane ring, alkyl, alkylidene, alkoxy, cycloalkyl, heterocycloalkyl, and the like described herein include both substituted and unsubstituted moiety, and possible substituent includes, but is not limited to, C₁-C₁₀ alkyl, C₂-C₁₀ alkenyl, C₂-C₁₀ alkynyl, C₃-C₂₀ cycloalkyl, C₃-C₂₀ cycloalkenyl, C₁-C₂₀ heterocycloalkyl, C₁-C₂₀ heterocycloalkenyl, C₁-C₁₀ alkoxy, aryl, aryloxy, heteroaryl, heteroaryloxy, amino, hydroxy, halogen, mercapto, cyano, nitro, carboxyl and carboxylate group.

### GPR84 antagonist

The GPR84 antagonist provided by the present invention is a compound having the structure of formula I shown above.

The invention also provides a pharmaceutically acceptable salt thereof, comprising a salt formed from the reaction of the compound of formula I and an inorganic or organic base compound. Salts derived from inorganic bases include, but are not limited to, aluminum salts, ammonium salts, calcium salts, copper salts, iron salts, ferrous salts, lithium salts, magnesium salts, manganese salts, manganite salts, potassium salts, sodium salts, zinc salts, etc. Ammonium salts, calcium salts, magnesium salts, potassium salts and sodium salts are particularly preferred. Salts are derived from pharmaceutically acceptable organic non-toxic bases including, but not limited to, salts of primary, secondary and tertiary amines, substituted amines include naturally occurring substituted amines, cyclic amines and basic ions exchange resin such as arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxycobalamin, isopropylamine, lysine, methyl glucosamine, morpholine, piperazine, piperidine, polyamine resin, procaine, purine, theobromine, triethylamine, trimethylamine, tripropylamine, trometamol, imidazole and the like.

### The main advantages of the present invention are as follow.

The inventors have developed a GPR84 antagonist with a novel structure. One ester group of the phosphodiester is a tricyclic segment, and the other ester group is derived into various structural types, such as (cyclo)alkyl or substituted (cyclo)alkyl , aryl or substituted aryl, cholic acid derivatives, etc. This modification helps to expand the chemical space of GPR84 antagonists, lowers CLogP, and makes them have better oral absorption and organ distribution characteristics, thus contributing to the treatment of GPR84-related diseases.

In summary, the compounds provided by the present invention can better maintain the antagonistic activity against GPR84, have better oral absorption, and have more specific target tissue distribution characteristics, thus having better development prospects.

### Preparation method

The compound of formula I can be prepared by any one of the following Route 1 to Route 5.

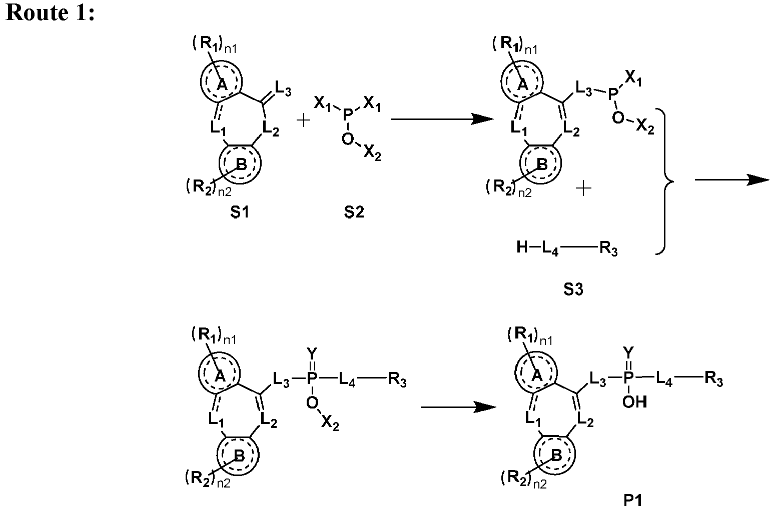

The first step reaction is carried out in dichloromethane or acetonitrile; the activating reagent used is 4,5-dicyanoimidazole or diisopropylaminotetrazolium salt or N-methylimidazole; the reaction temperature is 20°C to 60°C; the reaction time is about 1 to 24 hours; after the reaction is completed, the reaction mixture is neutralized with saturated NaHCO₃ solution, Na₂CO₃ solution, and extracted with AcOEt, Et₂O, CH₂Cl₂, CHCl₃ and other solvents, and the concentrate is purified by column chromatography. The second step reaction is carried out in dichloromethane or N,N-dimethylformamide, the activator used is tetrazole, the reaction time is about 1-24h, and then the oxidant is added to oxidize, the oxidant used is tert-butyl hydroperoxide or m-chloroperoxybenzoic acid, and the reaction time was about 0.3 to 2h. The reaction mixture is quenched by saturated Na₂SO₃, extracted with AcOEt, Et₂O, CH₂Cl₂, CHCl₃ and other solvents, and the concentrate was purified by column chromatography. The third step reaction is carried out in dichloromethane. Depending on the protective group X2, the conditions used were catalytic hydrogenation or alkali catalysis. The catalyst used for catalytic hydrogenation is Pd/C or Pd(OH)₂/C and hydrogen is added at atmospheric pressure; the base used for alkali catalysis is triethylamine or 1,8-diazabicyclohexadec-7-ene (DBU), and the reaction time is about 0.3-1 h. After completion of the reaction, the insoluble material is filtered off, and the filtrate is neutralized by addition of dilute hydrochloric acid, extracted with AcOEt, Et₂O, CH₂Cl₂, CHCl₃ and other solvents, and the concentrate was subjected to column chromatography to obtain the target product, which is confirmed by NMR and the like.

Wherein, R₁, R₂, R₃, L₁, L₂, L₄, L₅, Y, n1, n2, ring A and ring B are as defined above; L₃ is O; each X₁ is independently dimethylamino, ethylamino, diisopropylamino; X₂ is cyanoethyl, allyl, tert-butyl, benzyl.

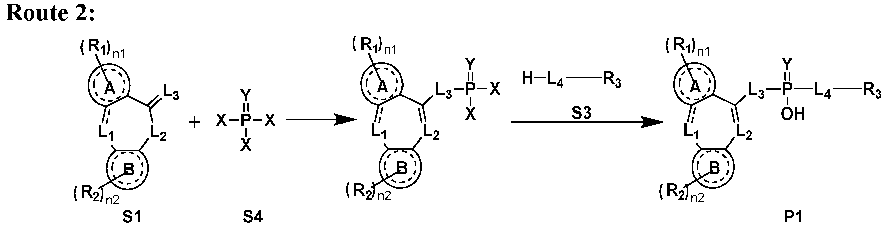

The reaction is carried out in pyridine; the reaction temperature is 60°C-100°C; the reaction time is about 1-24h; after the reaction is completed, the mixture is cooled to room temperature, S3 is added and reacted at 60°C-100°C for 1-24h, then water is added to quench the reaction. The mixture is extracted with AcOEt, Et₂O, CH₂Cl₂, CHCl₃ and other solvents, and the concentrate is purified by column chromatography to obtain the target product, which is confirmed by NMR and the like. Wherein, R₁, R₂, R₃, L₁, L₂, L₄, L₅, Y, n1, n2, ring A and ring B are as defined above; L₃ is O, S, NH or CH₂; each X is independently F, Cl, Br or I.

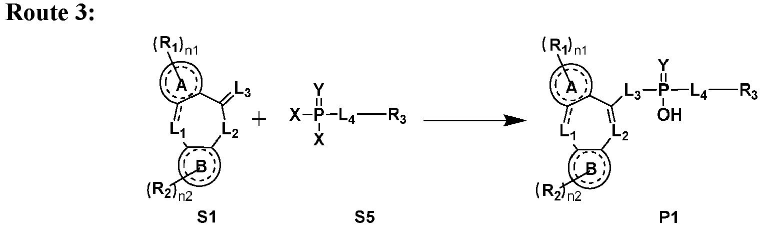

The reaction is carried out in pyridine; the reaction temperature is 60°C-100°C; the reaction time is about 1-24h; after the reaction is completed, the mixture is quenched with H₂O, extracted with AcOEt, Et₂O, CH₂Cl₂, CHCl₃ and other solvents, washed with saturated saline, and dried. The solvent is removed under reduced pressure at low temperature, and the concentrate is subjected to column chromatography to obtain the target product, which is confirmed by NMR and the like.

Wherein, R₁, R₂, R₃, L₁, L₂, L₄, L₅, Y, n1, n2, ring A and ring B are as defined above; L₃ is O or CH₂; each X is independently F, Cl, Br or I.

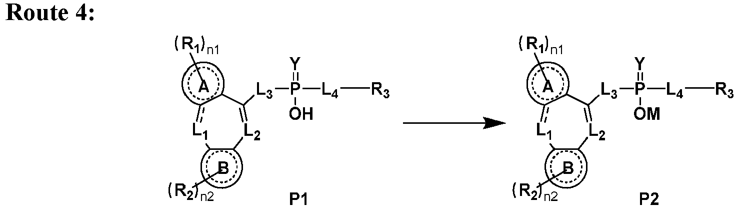

The raw material P1 is dissolved in ethyl acetate, and an aqueous solution of alkali (conjugate base of M, hydroxide of M or carbonate of M) is added to wash twice. The aqueous layer is back-extracted with ethyl acetate, and the ethyl acetate layer is concentrated. The crude product is chromatographed on a silica gel column to obtain the product P2.

M is a cation of the following metals: Li, Na, K, Ca, Mg, Cu, Fe, Zn, Al, Mn, or a conjugate acid of the following bases: NH₃, arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxycobalamin, isopropylamine, lysine, methyl glucosamine, morpholine, piperazine, piperidine, polyamine resin, procaine, purine, theobromine, triethylamine, trimethylamine, tripropylamine, trometamol, imidazole;
Wherein, R₁, R₂, R₃, L₁, L₂, L₄, L₅, Y, n1, n2, ring A and ring B are as defined above;
L₃ is O or CH₂.

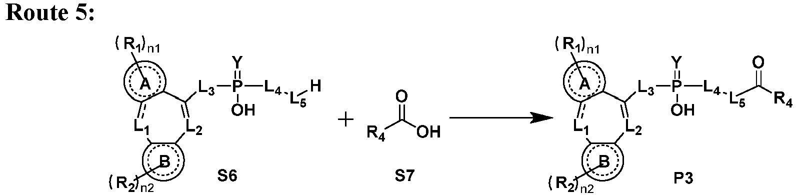

The reaction is carried out in N,N-dimethylformamide (DMF), and the activator used is 1-ethyl-(3-dimethylaminopropyl)carbodiimide hydrochloride (EDC·HCl), dimethylaminopyridine (DMAP), the base used is triethylamine or N,N-diisopropylethylamine, the reaction is carried out at room temperature for about 6 to 24 hours. After the reaction is completed, the solvent is removed under reduced pressure at low temperature, and the residue is extracted with ethyl acetate. The aqueous phase is back-extracted with water three times, and the mixture is washed with saturated brine, and the organic phase is concentrated and then subjected to column chromatography to obtain the target product, which is confirmed by NMR and the like.
Wherein, R₁, R₂, R₃, L₁, L₂, L₄, L₅, Y, n1, n2, ring A and ring B are as defined above;
L₅ is independently none, -(CH₂)ₘ-, -(CH₂)ₘ-CH=CH-, -(CH₂)ₘ-C ≡ C-, -(C₂H₄O)ₘ-, -(CH₂)ₘ-NH-, - (CH₂)ₘ-O-, each m is independently an integer of 0-10;
R₄ is substituted or unsubstituted C₁-C₁₀ alkyl, substituted or unsubstituted C₆-C₁₀ aromatic ring, C₃-C₁₀ cycloalkane ring, C₃-C₁₀ heterocycloalkane ring, C₃-C₁₀ heteroaryl ring, cholic acid, lithocholic acid, deoxycholic acid, isolithocholic acid, isodeoxycholic acid, chenodeoxycholic acid, ursodeoxycholic acid, α-muricholic acid, β-muricholic acid, γ-muricholic acid, ω-muricholic acid; the above substitution means there is one or more substituents selected from the following group consisting of C₁-C₆ alkyl, C₁-C₆ alkoxy, halogen, hydroxyl;
L₃ is O or CH₂.

### Use

As an antagonist of GPR84, the compound of formula I can competitively inhibit the activation of the receptor caused by the agonist of GPR84, and can be used for the preparation of a medicament for treating a disease caused by hyper-excitability or high expression of GPR84 receptor. Such diseases include multiple sclerosis, inflammatory bowel disease, organ fibrosis, arthritis, and the like.

### Pharmaceutical composition

The pharmaceutical composition of the present invention comprises a therapeutically effective amount of the compound of formula I or the pharmaceutically acceptable salt thereof, and one or more medicinal carriers.

"Medicinal carrier", "pharmaceutically acceptable carrier" or "pharmacologically available carrier" means one or more compatible solid or liquid filler or gel material which is suitable for human use and which must have sufficient purity and low toxicity. By "compatibility", it is meant herein that each component in the composition is capable of intermixing with the active ingredient of the present invention (the compound of formula I or a pharmaceutically acceptable salt thereof) without significantly reducing the efficacy of the active ingredient. Examples of pharmaceutically acceptable carriers are cellulose and its derivatives (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerin, mannitol, sorbitol, etc.), emulsifier (Tween^{®}), wetting agent (such as sodium dodecyl sulfate), colorant, flavoring agent, stabilizer, antioxidant, preservative, pyrogen-free water and the like.

The compounds and pharmaceutical compositions of the present invention may be in various forms, and may be administered orally in the form of, such as capsule, tablet, granule, solution, powder, pulvis or syrup, or administered in the non-oral form, such as an injection. The compounds and pharmaceutical compositions may be presented in a suitable solid or liquid vehicle and in a suitable sterilizing device for injection or drip. The above formulations can be prepared by conventional pharmaceutical methods.

The compounds and pharmaceutical compositions of the present invention are useful for clinical use in mammals, including humans and animals, and can be administered by the oral, nasal or gastrointestinal routes. The most preferred route of administration is oral.

The above features mentioned in the present invention, or the features mentioned in the embodiments, may be arbitrarily combined. All of the features disclosed in the present specification can be used in combination with any of the compositions, and each of the various features disclosed in the specification can be replaced by any alternative feature that provides the same, equal or similar purpose. Therefore, unless otherwise stated, the disclosed features are only general examples of equal or similar features.

The invention is further illustrated below in conjunction with specific examples. It is to be understood that the examples are for illustrative purposes only and are not intended to limit the scope of the invention. The experimental methods in the following examples which do not specify the specific conditions are usually carried out according to the conventional conditions, such as the conditions described in Sambrook et al., Molecular Cloning: Laboratory Manual (New York: Cold Spring Harbor Laboratory Press, 1989), or according to the conditions suggested by the manufacturer. Unless otherwise stated, percentages and parts are by weight.

In the following examples, NMR is measured with the AVANCE III 400M instrument produced by Bruker (NMR calibration: δ H 7.26 ppm (CDCl₃), 2.50 ppm (DMSO-*d₆*), 3.15 ppm (CD₃OD)). The reagents are mainly provided by Shanghai Chemical Reagent Co., Ltd.. TLC thin layer chromatography silica gel plate (model, HSGF 254) is produced by Shandong Yantai Huiyou Silicone Development Co., Ltd.. The normal phase column chromatography silica gel used for compound purification (model zcx- 11, 200-300 mesh) is produced by Branch Factory of Shandong Qingdao Marine Chemical Plant.

### Example 1

### Preparation of compound LSX448

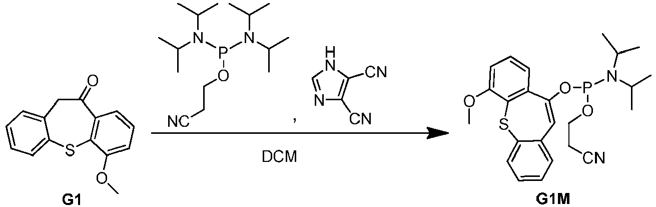

**Synthesis of intermediate G1M.** The starting material G1 (200 mg, 0.78 mmol), and 4,5-dicyanoimidazole (184 mg, 1.56 mmol) were dissolved in anhydrous dichloromethane, and bis(diisopropylamino )(2-cyanoethoxy)phosphine (0.495 ml, 1.56 mmol) was added dropwise under argon protection at 20°C, and reacted at 20°C for 5h. Then pH was adjusted to 7 with saturated NaHCO₃. The mixture was concentrated and extracted with dichloromethane. After the aqueous phase was back-extracted three times, the mixture was washed with saturated brine, dried over anhydrous Na₂SO₄, filtered, concentrated, and subjected to silica gel column chromatography (PE:AcOEt = 15:1-10:1) to obtain intermediate G1M (280 mg, 78%, colorless liquid).

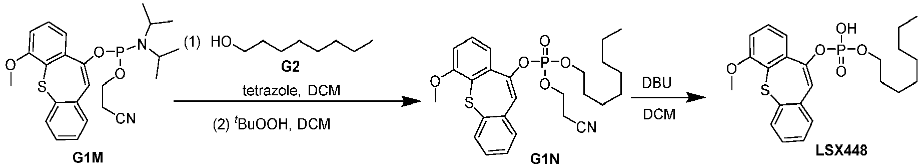

**Synthesis of Compound LSX448.** The intermediate G1M was dissolved in anhydrous dichloromethane, tetrazole (43 mg, 0.63 mmol) and G2 (0.097 ml, 0.63 mmol) were added and reacted for 4h. Then tert-butyl hydroperoxide (70% in water) (0.094 ml, 0.63 mmol) was added and reacted for 1h. The mixture was neutralized with saturated sodium sulfite solution, and extracted with ethyl acetate. After the aqueous phase was back-extracted three times, the mixture was washed with saturated brine, dried over anhydrous Na₂SO₄, filtered, concentrated, and subjected to silica gel column chromatography (PE:AcOEt = 10:1-2:1) to get the intermediate G1N. G1N was redissolved in anhydrous dichloromethane, and 1,8-diazabicycloundec-7-ene (0.094 ml, 0.63 mmol) was added and reacted for 30 mins. The mixture was adjusted to pH 7 with 1 mol/L HCl, and extracted with ethyl acetate. After the aqueous phase was back-extracted three times, the mixture was washed with saturated brine, dried over anhydrous Na₂SO₄, filtered, concentrated, and subjected to silica gel column chromatography (DCM:MeOH = 15:1-10:1), and dried by rotary evaporation to obtain the target compound LSX448 (35 mg, 10%, white solid). ¹H NMR (*d*₆-DMSO, 400 MHz): δ 7.46 (d, *J* = 7.2 Hz, 1H), 7.31-7.20 (m, 6H), 7.08 (d, *J* = 7.2 Hz, 1H), 3.87 (m, 2H), 3.83(s, 3H), 1.45(m, 2H), 1.24-1.14(m, 10H), 0.78(t, *J* = 7.6 Hz, 3H).

The following compounds were synthesized by the same method.

| No. | Structure formula | ¹H NMR data |
|---|---|---|
| LSX350 | 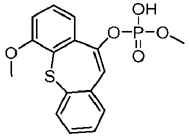 | ¹H NMR (400 MHz, DMSO-*d*₆) δ 7.46 (d, *J* = 7.6 Hz, 1H), 7.35 - 7.20 (m, 6H), 7.09 (d, *J* = 7.6 Hz, 1H), 3.84 (s, 3H), 3.49 (s, 3H). |
| LSX376 | 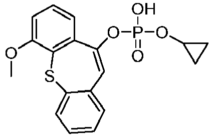 | ¹H NMR (400 MHz, DMSO-*d*₆) δ 7.46 (d, *J* = 7.6 Hz, 1H), 7.39 -7.15 (m, 6H), 7.09 (dd, *J* = 6.4, 3.2 Hz, 1H), 3.98 - 3.69 (m, 4H), 0.71 - 0.52 (m, 2H), 0.41 (d, *J* = 6.4 Hz, 2H). |
| LSX393 | 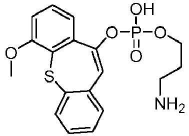 | ¹H NMR (400 MHz, DMSO-*d*₆) δ 7.50 (d, *J* = 7.6 Hz, 1H), 7.42 - 7.17 (m, 6H), 7.14 (d, *J* = 7.6 Hz, 1H), 4.03 (brs, 2H), 3.85 (s, 3H), 2.85 (brs, 2H), 2.09 (s, 2H), 1.90 (brs, 2H). |
| LSX406 | 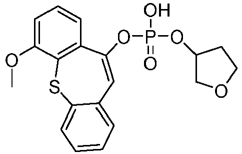 | ¹H NMR (400 MHz, DMSO-*d*₆) δ 7.51 (d, *J* = 7.2 Hz, 1H), 7.42 - 7.26 (m, 4H), 7.21 (d, *J* = 7.9 Hz, 1H), 7.18 - 7.13 (m, 2H), 5.03 - 4.87 (m, 1H), 3.78 - 3.58 (m, 4H), 2.11 - 1.82 (m, 2H). |
| LSX406-2 | 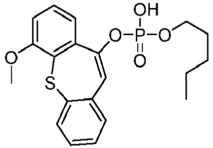 | ¹H NMR (400 MHz, DMSO-*d*₆) δ 7.45 (d, *J* = 7.6 Hz, 1H), 7.36 -7.14 (m, 6H), 7.10 - 7.01 (m, 1H), 3.82 (s, 3H), 3.80 - 3.71 (m, 2H), 1.54 - 1.38 (m, 2H), 1.27 - 1.15 (m, 4H), 0.78 (t, *J* = 7.0 Hz, 3H). |
| LSX412 | 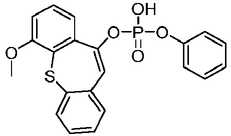 | ¹H NMR (400 MHz, DMSO-*d*₆) δ 7.50 (d, *J* = 7.2 Hz, 1H), 7.35-7.28 (m, 6H), 7.22-7.09 (m, 6H), 3.85 (s, 3H) |
| LSX418 | 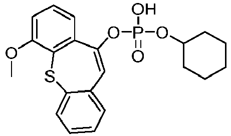 | ¹H NMR (400 MHz, DMSO-*d*₆) δ 7.49 - 7.40 (m, 1H), 7.36 - 7.14 (m, 6H), 7.06 (dd, *J* = 8.0, 1.6 Hz, 1H), 4.14 - 3.99 (m, 1H), 3.83 (s, 3H), 1.85 - 1.74 (m, 2H), 1.68 - 1.53 (m, 2H), 1.46 - 1.06 (m, 7H). |
| LSX420 | 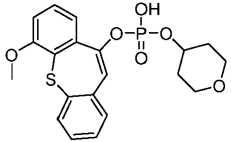 | ¹H NMR (400 MHz, DMSO-*d*₆) δ 7.51 (d, *J* = 7.2 Hz, 1H), 7.43 - 7.26 (m, 4H), 7.25 - 7.10 (m, 3H), 4.50 (tt, *J* = 8.6, 4.5 Hz, 1H), 3.85 (s, 3H), 3.77 - 3.65 (m, 2H), 3.43 - 3.26 (m, 2H), 1.90 - 1.74 (m, 2H), 1.56 (dtd, *J=* 12.8, 8.6, 3.8 Hz, 2H). |
| LSX420-3 | 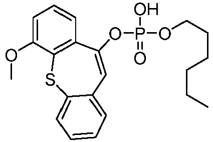 | ¹H NMR (400 MHz, DMSO-*d*₆) δ 7.46 (d, *J* = 7.5 Hz, 1H), 7.36 - 7.14 (m, 6H), 7.07 (dd, *J* = 5.9, 3.6 Hz, 1H), 3.83 (s, 3H), 3.80 - 3.73 (m, 2H), 1.51 - 1.39 (m, 2H), 1.28 - 1.11 (m, 6H), 0.78 (t, *J* = 7.2 Hz, 3H). |
| LSX426j | 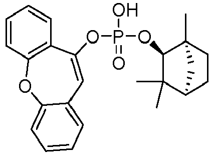 | ¹H NMR (400 MHz, DMSO-*d*₆) δ 7.78 - 7.66 (m, 1H), 7.51 - 7.43 (m, 2H), 7.42 - 7.34 (m, 3H), 7.34 - 7.27 (m, 1H), 7.23 (t, *J* = 7.5 Hz, 2H), 3.85 (d, *J* = 9.7 Hz, 1H), 1.80 - 1.67 (m, 1H), 1.67 - 1.55 (m, 2H), 1.46 (d, *J* = 9.9 Hz, 1H), 1.41 - 1.31 (m, 1H), 1.26 - 1.22 (m, 1H), 1.09 - 0.96 (m, 6H), 0.96 - 0.82 (m, 4H). |
| LSX430 | 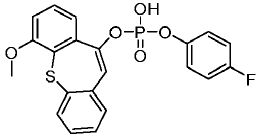 | ¹H NMR (400 MHz, DMSO-*d*₆) δ 7.49 (d, *J* = 7.2 Hz, 1H), 7.36 -7.04 (m, 11H), 3.84 (s, 3H). |
| LSX430-2 | 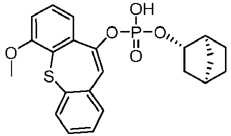 | ¹H NMR (400 MHz, DMSO-*d*₆) δ 7.49 (d, *J* = 7.2 Hz, 1H), 7.41 - 7.24 (m, 4H), 7.21 (d, *J* = 8.0 Hz, 1H), 7.19 - 7.09 (m, 2H), 4.23 (t, *J* = 7.2 Hz, 1H), 3.85 (s, 3H), 2.26 (d, *J* = 4.0 Hz, 1H), 2.21 - 2.13 (m, 1H), 1.63 - 1.49 (m, 1H), 1.49 - 1.25 (m, 4H), 1.10 - 0.89 (m, 3H). |
| LSX432-2a | 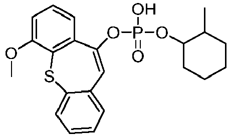 | ¹H NMR (400 MHz, DMSO-*d*₆) δ 7.47 (d, *J* = 7.6 Hz, 1H), 7.27 (m, 6H), 7.10 (d, *J* = 7.6 Hz, 1H), 3.84 (m, 4H), 1.70 - 1.09 (m, 10H), 0.94 - 0.80 (m, 3H). |
| LSX432-2b | 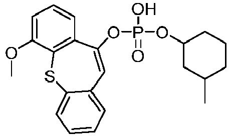 | ¹H NMR (400 MHz, DMSO-*d*₆) δ 7.46 (d, *J* = 7.6 Hz, 1H), 7.41 -7.13 (m, 6H), 7.08 (d, *J* = 7.6 Hz, 1H), 4.16 - 3.95 (m, 1H), 3.83 (s, 3H), 2.15 - 1.65 (m, 2H), 1.64 - 1.43 (m, 2H), 1.36 - 1.19 (m, 1H), 1.19 - 0.95 (m, 2H), 0.89 - 0.60 (m, 5H). |
| LSX432-2c | 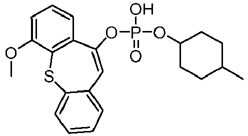 | ¹H NMR (400 MHz, DMSO-*d*₆) δ 7.45 (d, *J=* 7.6 Hz, 1H), 7.41 -7.15 (m, 6H), 7.08 (d, *J* = 7.6 Hz, 1H), 4.18 - 3.92 (m, 1H), 3.83 (s, 3H), 2.10 - 1.73 (m, 2H), 1.64 - 1.43 (m, 2H), 1.37 - 1.12 (m, 3H), 0.92 - 0.66 (m, 5H). |
| LSX432-3 | 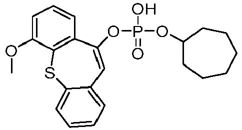 | ¹H NMR (400 MHz, DMSO-*d*₆) δ 7.46 (d, *J* = 7.6 Hz, 1H), 7.42 - 7.15 (m, 6H), 7.08 (d, *J* = 7.6 Hz, 1H), 4.53 - 4.22 (m, 1H), 3.83 (s, 3H), 1.96 - 1.77 (m, 2H), 1.66 - 1.13 (m, 12H). |
| LSX434 | 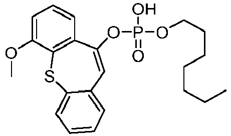 | ¹H NMR (400 MHz, DMSO-*d*₆) δ 7.46 (d, *J* = 7.6 Hz, 1H), 7.41 - 7.14 (m, 6H), 7.12 - 7.04 (m, 1H), 4.00 - 3.70 (m, 5H), 1.51 - 1.37 (m, 2H), 1.19 (s, 8H), 0.77 (t, 3H). |
| LSX434-2 | 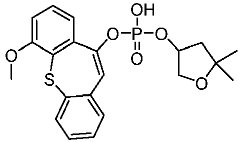 | ¹H NMR (400 MHz, DMSO-*d*₆) δ 7.51 (dd, *J* = 6.7, 1.5 Hz, 1H), 7.42 - 7.24 (m, 4H), 7.24 - 7.09 (m, 3H), 4.58 - 4.45 (m, 1H), 3.85 (s, 3H), 3.62 (ddd, *J* = 12.2, 5.0, 3.1 Hz, 1H), 3.47 (td, *J* = 11.8, 2.6 Hz, 1H), 1.87 - 1.72 (m, 2H), 1.51- 1.35 (m, 1H), 1.30 (dd, *J* = 12.5, 10.4 Hz, 1H), 1.10 (s, 3H), 1.05 (s, 3H). |
| LSX442a | 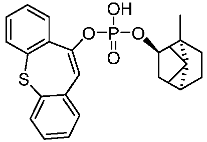 | ¹H NMR (400 MHz, DMSO-*d*₆) δ 7.67 (d, *J* = 5.6 Hz, 1H), 7.48 - 7.40 (m, 2H), 7.42 - 7.32 (m, 2H), 7.32 - 7.25 (m, 2H), 7.25 - 7.17 (m, 2H), 4.37 (t, *J* = 9.1 Hz, 1H), 2.13 - 1.91 (m, 2H), 1.68 - 1.56 (m, 1H), 1.56 - 1.48 (m, 1H), 1.15 - 1.09 (m, 3H), 0.88 - 0.70 (m, 9H). |
| LSX442j | 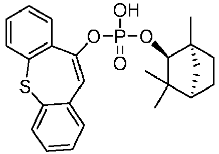 | ¹H NMR (400 MHz, DMSO-*d*₆) δ 7.77 - 7.64 (m, 1H), 7.53 - 7.41 (m, 2H), 7.40 - 7.32 (m, 3H), 7.32 - 7.25 (m, 1H), 7.25 - 7.16 (m, 2H), 3.82 (d, *J* = 9.8 Hz, 1H), 1.80 - 1.69 (m, 1H), 1.69 - 1.56 (m, 2H), 1.46 (d, *J* = 10.0 Hz, 1H), 1.41 - 1.28 (m, 1H), 1.07 - 0.94 (m, 7H), 0.94 - 0.81 (m, 4H). |
| LSX444 | 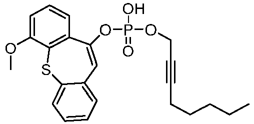 | ¹H NMR (400 MHz, DMSO-*d*₆) δ 7.44 (d, *J* = 7.2 Hz, 1H), 7.35 - 7.14 (m, 6H), 7.07 (d, *J* = 7.2 Hz, 1H), 4.39 (s, 2H), 3.83 (s, 3H), 2.09 (d, *J* = 8.4 Hz, 2H), 1.34-1.14 (m, 6H), 0.83 - 0.78 (m, 3H). |
| LSX444-2 | 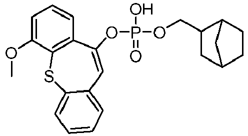 | ¹H NMR (400 MHz, DMSO-*d*₆) δ 7.47 (d, *J* = 7.6 Hz, 1H), 7.41 -7.16 (m, 6H), 7.11 (d, *J* = 7.6 Hz, 1H), 3.84 (s, 3H), 3.70 (d, *J* = 6.8 Hz, 1H), 3.54 (d, *J* = 6.8 Hz, 1H), 2.20 - 2.12 (m, 1H), 2.12 - 1.97 (m, 2H), 1.65 - 1.47 (m, 1H), 1.47 - 1.35 (m, 2H), 1.11- 0.90 (m, 2H), 0.90 - 0.81 (m, 2H), 0.56 (d, *J* = 9.2 Hz, 1H). |
| LSX446 | 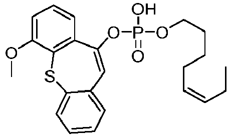 | ¹H NMR (400 MHz, DMSO-*d*₆) δ 7.53 - 7.15 (m, 7H), 7.13 - 7.05 (m, 1H), 5.35 - 5.03 (m, 2H), 3.99 - 3.76 (m, 5H), 1.96 - 1.73 (m, 4H), 1.52 - 1.41 (m, 2H), 1.19 - 1.10 (m, 2H), 0.84 - 0.78 (m, 3H). |
| LSX446-2 | 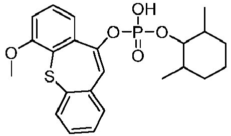 | ¹H NMR (400 MHz, DMSO-*d*₆) δ 7.51 - 7.41 (m, 1H), 7.39 -7.13 (m, 6H), 7.10 - 7.02 (m, 1H), 3.83 (s, 3H), 3.53 (q, *J* = 10.0 Hz, 1H), 1.64 (d, *J* = 13.2 Hz, 2H), 1.45 - 1.29 (m, 4H), 1.01 (d, *J* = 6.8 Hz, 6H), 0.89 (dd, *J* = 6.8, 4.2 Hz, 2H). |
| LSX452 | 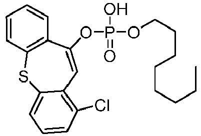 | ¹H NMR (400 MHz, DMSO-*d*₆) δ 7.66 (d, *J* = 7.2 Hz, 1H), 7.60 - 7.32 (m, 6H), 7.25 (d, *J* = 7.2 Hz, 1H), 3.88 - 3.67 (m, 2H), 1.53 - 1.39 (m, 2H), 1.28 - 1.13 (m, 10H), 0.82 - 0.77 (m, 3H). |
| LSX452-2 | 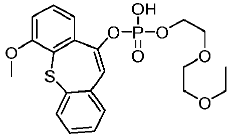 | ¹H NMR (400 MHz, DMSO-*d*₆) δ 7.46 (d, *J* = 7.6 Hz, 1H), 7.36 - 7.18 (m, 7H), 7.09 (dd, *J* = 7.6, 2.4 Hz, 1H), 4.04 - 3.88 (m, 2H), 3.84 (s, 3H), 3.51 (t, *J* = 5.2 Hz, 2H), 3.49 - 3.22 (m, 6H), 1.04 (t, *J* = 7.2 Hz, 3H). |
| LSX454 | 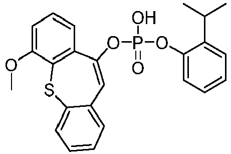 | ¹H NMR (400 MHz, DMSO-*d*₆) δ 7.52 (d, *J* = 8.0 Hz, 1H), 7.45 (d, *J* = 7.6 Hz, 1H), 7.34 - 7.11 (m, 6H), 7.06 (d, *J* = 8.0 Hz, 2H), 6.95 (t, *J* = 7.6 Hz, 1H), 3.83 (s, 3H), 3.43 - 3.38 (m, 1H), 1.06 (d, *J* = 6.7 Hz, 6H). |
| LSX456j | 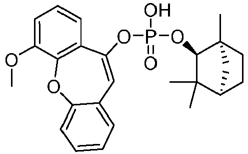 | ¹H NMR (400 MHz, DMSO-*d*₆) δ 7.24 - 7.04 (m, 7H), 6.97 (d, *J* = 12.7 Hz, 1H), 3.86 (s, 3H), 3.77 (d, *J* = 9.9 Hz, 1H), 1.78 - 1.68 (m, 1H), 1.68 - 1.55 (m, 2H), 1.44 (d, *J* = 9.9 Hz, 1H), 1.40 - 1.26 (m, 2H), 1.05 - 0.93 (m, 7H), 0.91 - 0.78 (m, 4H). |
| LSX457 | 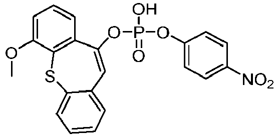 | ¹H NMR (400 MHz, DMSO-*d*₆) δ 8.16 - 8.10 (m, 2H), 7.52 - 7.35 (m, 3H), 7.35 - 7.17 (m, 5H), 7.13 (dd, *J* = 8.0, 1.2 Hz, 1H), 7.05 (dd, *J* = 8.0, 1.2 Hz, 1H), 3.81 (s, 3H). |
| LSX460 | 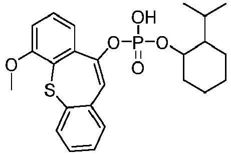 | ¹H NMR (400 MHz, DMSO-*d*₆) δ 7.48 (d, *J* = 7.6 Hz, 1H), 7.42 -7.17 (m, 6H), 7.12 (d, *J* = 7.6 Hz, 1H), 4.72 - 4.59 (m, 1H), 3.84 (s, 3H), 2.12 (d, *J* = 13.2 Hz, 1H), 1.59 - 1.43 (m, 3H), 1.27 (d, *J* = 24.0 Hz, 2H), 1.19 - 1.08 (m, 2H), 1.01 - 0.73 (m, 8H). |
| LSX460a | 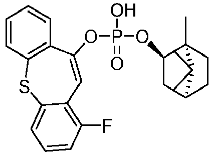 | ¹H NMR (400 MHz, DMSO-*d*₆) δ 7.69 - 7.62 (m, 1H), 7.49 - 7.24 (m, 6H), 7.20 - 7.09 (m, 1H), 4.40 (t, *J* = 9.0 Hz, 1H), 2.14 - 1.94 (m, 2H), 1.67 - 1.55 (m, 1H), 1.54 - 1.47 (m, 1H), 1.14 - 1.09 (m, 3H), 0.83 - 0.73 (m, 9H). |
| LSX460j | 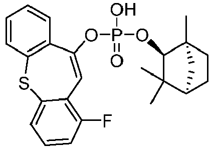 | ¹H NMR (400 MHz, DMSO-*d*₆) δ 7.75 - 7.66 (m, 1H), 7.50 - 7.45 (m, 1H), 7.44 - 7.37 (m, 3H), 7.33 - 7.22 (m, 2H), 7.20 - 7.11 (m, 1H), 3.86 (dd, *J* = 9.8, 1.7 Hz, 1H), 1.82 - 1.70 (m, 1H), 1.69 - 1.55 (m, 2H), 1.51 - 1.41 (m, 1H), 1.35 (tdd, *J* = 12.4, 5.9, 4.0 Hz, 1H), 1.08 - 0.94 (m, 7H), 0.94 - 0.83 (m, 4H). |
| LSX462a | 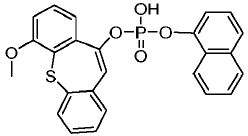 | ¹H NMR (400 MHz, Methanol-*d*₄) δ 8.06 (d, *J* = 8.0 Hz, 1H), 7.79 - 7.63 (m, 2H), 7.44 (t, *J* = 8.0 Hz, 2H), 7.38 - 7.21 (m, 4H), 7.14 - 6.98 (m, 4H), 6.85 (t, *J* = 7.6 Hz, 1H), 6.67 (d, *J* = 7.6 Hz, 1H), 3.76 (s, 3H). |
| LSX462b | 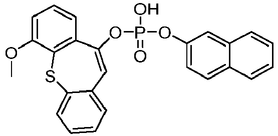 | ¹H NMR (400 MHz, DMSO-*d*₆) δ 7.83-7.71 (m, 4H), 7.47-7.15 (m, 10H), 7.02 (d, *J* = 7.2 Hz, 1H), 3.81(s, 3H) |
| LSX462-2 | 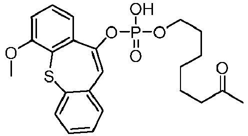 | ¹H NMR (400 MHz, DMSO-*d*₆) δ 7.47 (d, *J* = 7.6 Hz, 1H), 7.44 - 7.17 (m, 6H), 7.09 (dd, *J* = 7.6, 2.0 Hz, 1H), 4.01 - 3.77 (m, 5H), 2.31 - 2.14 (m, 2H), 1.98 (s, 3H), 1.50 - 1.37 (m, 2H), 1.37 - 1.01 (m, 6H). |
| LSX462-3 | 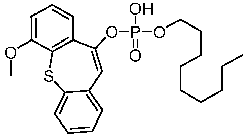 | ¹H NMR (400 MHz, DMSO-*d*₆) δ 7.46 (d, *J* = 7.6 Hz, 1H), 7.40 -7.14 (m, 6H), 7.11 - 7.03 (m, 1H), 3.99 - 3.71 (m, 5H), 1.53 - 1.37 (m, 2H), 1.22 - 0.98 (m, 12H), 0.80 (t, *J* = 7.2 Hz, 3H). |
| LSX464 | 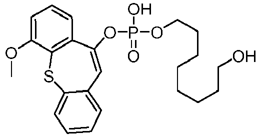 | ¹H NMR (400 MHz, DMSO-*d*₆) δ 7.44 (d, *J* = 7.2 Hz, 1H), 7.34 - 7.14 (m, 6H), 7.07 (d, *J* = 7.2 Hz, 1H), 4.32 (brs, 1H), 3.83 (s, 3H), 3.78 - 3.63 (m, 2H), 1.52 - 1.11 (m, 14H). |
| LSX466 | 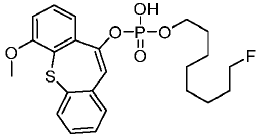 | ¹H NMR (400 MHz, DMSO-*d*₆) δ 7.46 (d, *J* = 7.6 Hz, 1H), 7.41 - 7.14 (m, 6H), 7.09 (dd, *J* = 7.6, 2.8 Hz, 1H), 4.38 (t, *J* = 6.0 Hz, 1H), 4.27 (t, *J* = 6.0 Hz, 1H), 3.99 - 3.77 (m, 5H), 1.59 - 1.41 (m, 4H), 1.33 - 0.95 (m, 8H). |
| LSX468a | 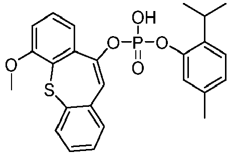 | ¹H NMR (400 MHz, DMSO-*d*₆) δ 7.46 (d, *J* = 7.6 Hz, 1H), 7.37 - 7.12 (m, 7H), 7.05 (dd, *J* = 15.6, 7.6 Hz, 2H), 6.77 (d, *J* = 7.6 Hz, 1H), 3.83 (s, 3H), 2.19 (s, 3H), 1.04 (d, *J* = 6.8 Hz, 6H). |
| LSX468b | 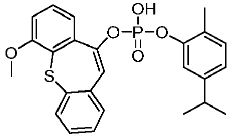 | ¹H NMR (400 MHz, DMSO-*d*₆) δ 7.46 (d, *J* = 8.0 Hz, 1H), 7.37 (s, 1H), 7.28 (t, *J* = 7.2 Hz, 2H), 7.26 - 7.15 (m, 3H), 7.12 (d, *J* = 7.2 Hz, 1H), 7.06 (d, *J* = 8.0 Hz, 1H), 6.99 (d, *J* = 7.6 Hz, 1H), 6.77 (d, *J* = 8.0 Hz, 1H), 3.83 (s, 3H), 2.72 (p, *J* = 6.8 Hz, 1H), 1.08 (d, *J* = 6.8 Hz, 6H). |
| LSX470a | 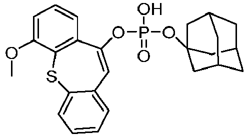 | ¹H NMR (400 MHz, DMSO-*d*₆) δ 7.45 (d, *J* = 7.2 Hz, 1H), 7.36 -7.12 (m, 5H), 7.07 (d, *J* = 7.2 Hz, 1H), 3.83 (s, 3H), 2.13 - 1.88 (m, 9H), 1.61 - 1.41 (m, 6H). |
| LSX470b | 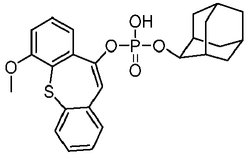 | ¹H NMR (400 MHz, DMSO-*d*₆) δ 7.45 (d, *J* = 7.6 Hz, 1H), 7.37 - 7.25 (m, 3H), 7.26 - 7.15 (m, 3H), 7.07 (d, *J* = 7.6 Hz, 1H), 3.83 (s, 3H), 2.06 (d, *J* = 12.0 Hz, 2H), 1.94 (s, 2H), 1.76 - 1.66 (m, 4H), 1.67 - 1.56 (m, 4H), 1.43 - 1.34 (m, 2H). |
| LSX472a | 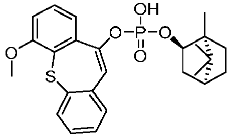 | ¹H NMR (400 MHz, DMSO-*d*₆) δ 7.50 (d, *J* = 7.2 Hz, 1H), 7.37-7.29 (m, 4H), 7.25 - 7.10 (m, 3H), 4.49 (d, *J* = 9.2 Hz, 1H), 3.85 (s, 3H), 2.20 - 2.09 (m, 1H), 1.85-1.82 (m, 1H), 1.71 - 1.54 (m, 2H), 1.19-1.11 (m, 3H), 0.83 - 0.77 (m, 9H). |
| LSX472b | 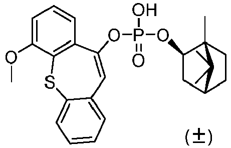 | ¹H NMR (400 MHz, DMSO-*d*₆) δ 7.47 - 7.40 (d, *J* = 7.2, 1H), 7.38 - 7.11 (m, 6H), 7.06 (d, *J* = 7.2, 1H), 4.09 (td, *J* = 8.4, 3.6 Hz, 1H), 3.83 (s, 3H), 1.85 (dt, *J* = 12.0, 3.6 Hz, 1H), 1.62 - 1.51 (m, 3H), 1.48 - 1.37 (m, 1H), 1.00 - 0.94 (m, 5H), 0.83 (s, 3H), 0.76 (s, 3H). |
| LSX472c | 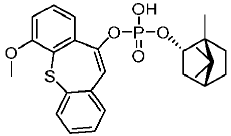 | ¹H NMR (400 MHz, DMSO-*d*₆) δ 7.48 (d, *J* = 7.6 Hz, 1H), 7.38 -7.19 (m, 6H), 7.11 (d, *J* = 7.6 Hz, 1H), 4.57 - 4.32 (m, 1H), 3.84 (s, 3H), 2.18 - 2.06 (m, 1H), 1.96 - 1.83 (m, 1H), 1.69 - 1.59 (m, 1H), 1.59 - 1.52 (m, 1H), 1.20 - 1.06 (m, 3H), 0.83 - 0.74 (m, 9H). |
| LSX472d | 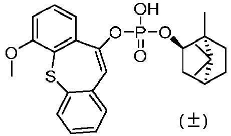 | ¹H NMR (400 MHz, DMSO-*d*₆) δ 7.46 (d, *J* = 7.6 Hz, 1H), 7.38 - 7.15 (m, 6H), 7.07 (d, *J* = 7.6 Hz, 1H), 4.50 - 4.33 (m, 1H), 3.83 (s, 3H), 2.13 - 1.93 (m, 1H), 1.67 - 1.56 (m, 1H), 1.56 - 1.48 (m, 1H), 1.33 - 1.20 (m, 1H), 1.19 - 1.05 (m, 3H), 0.86 - 0.71 (m, 9H). |
| LSX472e | 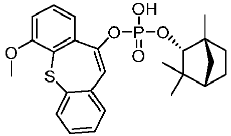 | ¹H NMR (400 MHz, DMSO-*d*₆) δ 7.46 (d, *J* = 7.2 Hz, 1H), 7.41 -7.14 (m, 6H), 7.07 (d, *J* = 7.2 Hz, 1H), 3.91 - 3.74 (m, 4H), 1.79 - 1.53 (m, 3H), 1.50 - 1.45 (m, 1H), 1.43 - 1.29 (m, 1H), 1.29 - 1.18 (m, 1H), 1.05 - 0.98 (m, 7H), 0.93 - 0.84 (m, 3H). |
| LSX472f | 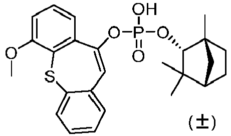 | ¹H NMR (400 MHz, DMSO-*d*₆) δ 7.44 (d, *J* = 7.6 Hz, 1H), 7.39 - 7.24 (m, 4H), 7.19 (t, *J* = 7.6 Hz, 2H), 7.10 - 7.02 (m, 1H), 3.89 - 3.76 (m, 4H), 1.80 - 1.67 (m, 1H), 1.67 - 1.54 (m, 2H), 1.51 - 1.42 (m, 1H), 1.37 - 1.31 (m, 1H), 1.23 - 1.22 (m, 1H), 1.04 - 0.97 (m, 7H), 0.89 (s, 3H). |
| LSX472g | 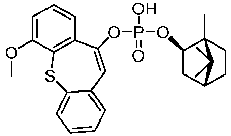 | ¹H NMR (400 MHz, DMSO-*d*₆) δ 7.45 (d, *J* = 7.6 Hz, 1H), 7.39 -7.13 (m, 6H), 7.06 (d, *J* = 7.6 Hz, 1H), 4.11 (t, *J* = 8.4 Hz, 1H), 3.83 (s, 3H), 1.96 - 1.81 (m, 1H), 1.67 - 1.51 (m, 3H), 1.48 - 1.41 (m, 1H), 1.08 - 0.90 (m, 5H), 0.84 (s, 3H), 0.76 (s, 3H). |
| LSX472h | 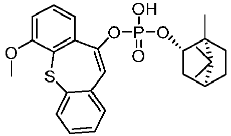 | ¹H NMR (400 MHz, DMSO-*d*₆) δ 7.45 (d, *J* = 7.6 Hz, 1H), 7.42 - 7.16 (m, 6H), 7.08 (d, *J* = 7.6 Hz, 1H), 4.14 t, *J* = 8.4 Hz, 1H), 3.84 (s, 3H), 1.96 - 1.81 (m, 1H), 1.69 - 1.54 (m, 3H), 1.49 - 1.36 (m, 1H), 1.05 - 0.89 (m, 5H), 0.84 (s, 3H), 0.76 (s, 3H). |
| LSX472j | 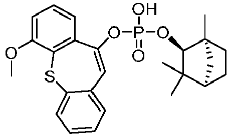 | ¹H NMR (400 MHz, DMSO-*d*₆) δ 7.50 - 7.41 (m, 1H), 7.37 (d, *J* = 7.2 Hz, 1H), 7.33 - 7.24 (m, 3H), 7.24 - 7.16 (m, 2H), 7.06 (dd, *J* = 6.0, 3.6 Hz, 1H), 3.93 - 3.75 (m, 4H), 1.81 - 1.68 (m, 1H), 1.68 - 1.52 (m, 2H), 1.46 (d, *J* = 10.0 Hz, 1H), 1.42 - 1.25 (m, 2H), 1.08 - 0.96 (m, 7H), 0.92 - 0.83 (m, 4H). |
| LSX474a | 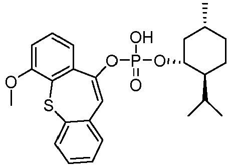 | ¹H NMR (400 MHz, DMSO-*d*₆) δ 7.48 - 7.39 (m, 1H), 7.36 - 7.12 (m, 6H), 7.05 (dd, *J* = 7.6, 2.0 Hz, 1H), 3.85 - 3.83 (m, 4H), 2.34 - 2.19 (m, 2H), 1.63 - 1.49 (m, 2H), 1.37 - 1.26 (m, 2H), 1.15 - 1.03 (m, 1H), 0.95 - 0.89 (m, 1H), 0.80 (dd, *J* = 13.6, 6.8 Hz, 6H), 0.67 (d, *J* = 6.8 Hz, 3H). |
| LSX474b | 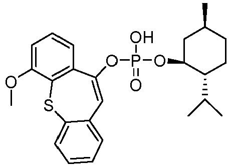 | ¹H NMR (400 MHz, DMSO-*d*₆) δ 7.51 - 7.39 (m, 1H), 7.36 - 7.22 (m, 4H), 7.18 (t, *J* = 7.6 Hz, 2H), 7.05 (dd, *J* = 7.2, 2.0 Hz, 1H), 3.95 - 3.77 (m, 4H), 2.36 - 2.20 (m, 2H), 1.64 - 1.49 (m, 2H), 1.37 - 1.23 (m, 2H), 1.16-1.07 (m, 1H), 0.96 - 0.82 (m, 1H), 0.80 (dd, *J* = 13.2, 6.8 Hz, 6H), 0.67 (d, *J =* 6.8 Hz, 3H). |
| LSX474-2 | 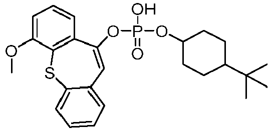 | ¹H NMR (400 MHz, DMSO-*d*₆) δ 7.45 (d, *J* = 7.6 Hz, 1H), 7.38 - 7.17 (m, 6H), 7.08 (d, *J* = 7.6 Hz, 1H), 4.17 - 3.92 (m, 1H), 3.83 (s, 3H), 2.21 - 1.95 (m, 2H), 1.78 - 1.44 (m, 2H), 1.22 - 1.08 (m, 2H), 0.97 - 0.66 (m, 12H). |
| LSX474-3 | 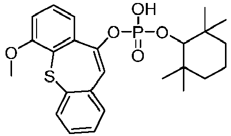 | ¹H NMR (400 MHz, DMSO-*d*₆) δ 7.44 (dd, *J* = 7.6, 1.6 Hz, 1H), 7.36 - 7.22 (m, 4H), 7.22 - 7.10 (m, 2H), 7.05 (dd, *J* = 7.6, 1.6 Hz, 1H), 3.83 (s, 3H), 3.77 (d, *J* = 10.8 Hz, 1H), 1.42 - 1.35 (m, 2H), 1.32 - 1.21 (m, 2H), 1.22 - 1.10 (m, 2H), 0.98 (s, 6H), 0.90 (s, 6H). |
| LSX476a | 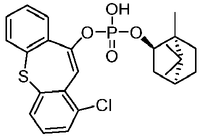 | ¹H NMR (400 MHz, DMSO-*d*₆) δ 7.68 - 7.62 (m, 1H), 7.50 - 7.35 (m, 6H), 7.22 (t, *J* = 7.6 Hz, 1H), 4.42 - 4.39 (m, 1H), 2.10 - 1.95 (m, 1H), 1.70 - 1.55 (m, 1H), 1.55 - 1.48 (m, 1H), 1.22 - 1.06 (m, 3H), 0.84 - 0.73 (m, 9H). |
| LSX476b | 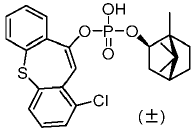 | ¹H NMR (400 MHz, DMSO-*d*₆) δ 7.73 - 7.59 (m, 1H), 7.59 - 7.36 (m, 6H), 7.24 (t, *J* = 7.6 Hz, 1H), 4.24 - 4.12 (m, 1H), 1.93 - 1.83 (m, 1H), 1.65 - 1.55 (m, 3H), 1.42 (t, *J =* 10.0 Hz, 1H), 1.05 - 0.90 (m, 5H), 0.82 (s, 3H), 0.75 (s, 3H). |
| LSX476c | 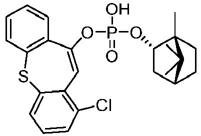 | ¹H NMR (400 MHz, DMSO-*d*₆) δ 7.67 - 7.61 (m, 1H), 7.50 - 7.35 (m, 6H), 7.22 (t, *J* = 7.6 Hz, 1H), 4.40 (td, *J* = 9.2, 4.8 Hz, 1H), 2.12 - 1.97 (m, 1H), 1.70 - 1.55 (m, 1H), 1.55 - 1.49 (m, 1H), 1.22 - 1.06 (m, 3H), 0.84 - 0.73 (m, 9H). |
| LSX476d | 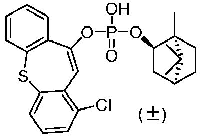 | ¹H NMR (400 MHz, DMSO-*d*₆) δ 7.67 - 7.61 (m, 1H), 7.50 - 7.35 (m, 6H), 7.22 (t, *J* = 7.6 Hz, 1H), 4.40 (td, *J* = 9.2, 4.8 Hz, 1H), 2.12 - 1.97 (m, 1H), 1.70 - 1.55 (m, 1H), 1.55 - 1.49 (m, 1H), 1.22 - 1.06 (m, 3H), 0.84 - 0.73 (m, 9H). |
| LSX476e | 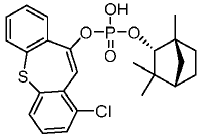 | ¹H NMR (400 MHz, DMSO-*d*₆) δ 7.72 - 7.63 (m, 1H), 7.59 - 7.47 (m, 5H), 7.44 - 7.31 (m, 2H), 3.96 (d, *J* =10.0 Hz, 1H), 1.71 - 1.48 (m, 4H), 1.47 - 1.31 (m, 1H), 1.11 (d, *J* = 10.0 Hz, 1H), 1.08 - 0.91 (m, 7H), 0.86 (s, 3H). |
| LSX476f | 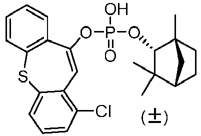 | ¹H NMR (400 MHz, DMSO-*d*₆) δ 7.70 - 7.63 (m, 1H), 7.56 - 7.34 (m, 6H), 7.21 (t, *J* = 7.6 Hz, 1H), 3.83 (d, *J* = 10.0 Hz, 1H), 1.84 - 1.72 (m, 1H), 1.68 - 1.55 (m, 2H), 1.45 (d, *J* = 10.0 Hz, 1H), 1.39 - 1.23 (m, 2H), 1.06 - 0.95 (m, 7H), 0.92 - 0.85 (m, 4H). |
| LSX476g | 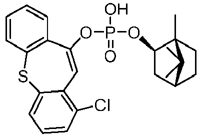 | ¹H NMR (400 MHz, DMSO-*d*₆) δ 7.73 - 7.61 (m, 1H), 7.61 - 7.44 (m, 5H), 7.44 - 7.31 (m, 2H), 3.96 (dd, *J* = 8.8, 1.6 Hz, 1H), 1.69 - 1.49 (m, 4H), 1.39 (ddt, *J* = 16.4, 6.8, 3.6 Hz, 1H), 1.11 (d, *J* = 10.0 Hz, 1H), 1.09-0.96 (m, 7H), 0.86 (s, 3H). |
| LSX476h | 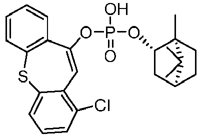 | ¹H NMR (400 MHz, DMSO-*d*₆) δ 7.71 - 7.61 (m, 1H), 7.61 - 7.44 (m, 5H), 7.40 - 7.31 (m, 2H), 3.96 (dd, *J* = 9.1, 1.7 Hz, 1H), 1.70 - 1.46 (m, 4H), 1.38 (ddt, *J* = 13.2, 7.2, 3.6 Hz, 1H), 1.12 (d, *J* = 10.0 Hz, 1H), 1.05 - 0.92 (m, 7H), 0.85 (s, 3H). |
| LSX476j | 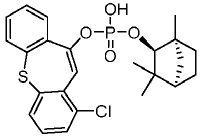 | ¹H NMR (400 MHz, DMSO-*d*₆) δ 7.72 - 7.61 (m, 1H), 7.61 - 7.47 (m, 5H), 7.43 - 7.32 (m, 2H), 3.96 (d, *J* = 10.0 Hz, 1H), 1.71 - 1.47 (m, 4H), 1.46 - 1.30 (m, 1H), 1.12 (d, *J* = 10.0 Hz, 1H), 1.08 - 0.91 (m, 7H), 0.85 (s, 3H). |
| LSX478 | 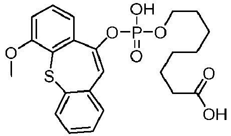 | ¹H NMR (400 MHz, DMSO-*d*₆) δ 7.45 (d, *J* = 7.2 Hz, 1H), 7.42 -7.14 (m, 6H), 7.06 (d, *J* = 7.2 Hz, 1H), 3.83 (s, 3H), 3.73 (d, *J* = 6.4 Hz, 2H), 2.19 - 2.09 (m, 2H), 1.49 - 1.40 (m, 4H), 1.27 - 1.16 (m, 6H). |
| LSX480 | 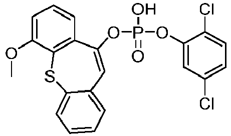 | ¹H NMR 400 MHz, DMSO-*d*₆) δ 7.85 (s, 1H), 7.49 - 7.11 (m, 8H), 7.06 (d, *J* = 8.4 Hz, 2H), 3.83 (s, 3H). |
| LSX482a | 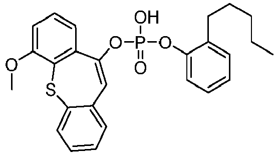 | ¹H NMR (400 MHz, DMSO-*d*₆) δ 7.65 - 7.49 (m, 1H), 7.45 (d, *J* = 7.6 Hz, 1H), 7.41 - 6.96 (m, 9H), 6.90 (t, *J* = 7.6 Hz, 1H), 3.82 (s, 3H), 2.62 - 2.42 (m, 2H), 1.54 - 1.41 (m, 2H), 1.23 - 0.97 (m, 4H), 0.78 - 0.59 (m, 3H). |
| LSX482b | 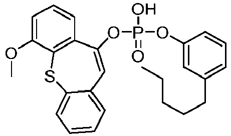 | ¹H NMR (400 MHz, DMSO-*d*₆) δ 7.46 (d, *J* = 7.6 Hz, 1H), 7.35 - 6.97 (m, 10H), 6.80 (d, *J* = 7.6 Hz, 1H), 3.82 (s, 3H), 2.44 (t, *J* = 7.6 Hz, 2H), 1.45 - 1.40 (m, 2H), 1.26 - 1.15 (m, 2H), 0.86 - 0.79 (m, 5H). |
| LSX482c | 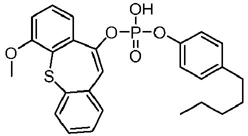 | ¹H NMR (400 MHz, DMSO-*d*₆) δ 7.46 (dd, *J* = 8.0, 1.2 Hz, 1H), 7.34 - 7.26 (m, 2H), 7.26 -7.15 (m, 3H), 7.15 - 6.97 (m, 6H), 3.82 (s, 3H), 2.47 (t, *J* = 7.6 Hz, 2H), 1.51 (p, *J* = 7.6 Hz, 2H), 1.38 - 1.18 (m, 4H), 0.84 (t, *J* = 6.8 Hz, 3H). |
| LSX491a | 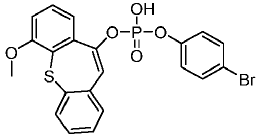 | ¹H NMR (400 MHz, DMSO-*d*₆) δ 7.46 (d, *J* = 7.6 Hz, 1H), 7.39 (d, *J* = 8.4 Hz, 2H), 7.33 - 7.16 (m, 7H), 7.08 (d, *J* = 7.6 Hz, 2H), 3.83 (s, 3H). |
| LSX492 | 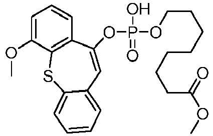 | ¹H NMR (400 MHz, DMSO-*d*₆) δ 7.50 - 7.17 (m, 7H), 7.08 (dd, *J* = 7.2, 3.2 Hz, 1H), 3.96 - 3.73 (m, 5H), 3.56 (s, 3H), 2.17 (t, *J* = 7.6 Hz, 2H), 1.46 - 1.34 (m, 4H), 1.21 - 1.04 (m, 6H). |
| LSX496 | 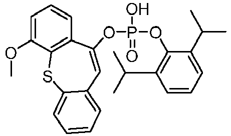 | ¹H NMR (400 MHz, DMSO-*d*₆) δ 7.55 - 7.41 (m, 1H), 7.36 (s, 1H), 7.34 - 7.25 (m, 3H), 7.25 - 7.14 (m, 2H), 7.08 (q, *J* = 4.4 Hz, 1H), 7.04 - 6.92 (m, 3H), 3.85 (s, 3H), 3.76 (p, *J* = 6.8 Hz, 2H), 1.07 (d, *J* = 6.8 Hz, 12H). |
| LSX498 | 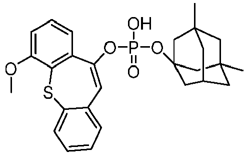 | ¹H NMR (400 MHz, DMSO-*d*₆) δ 7.48 - 7.43 (m, 1H), 7.29 (dd, *J* = 8.8, 5.2 Hz, 4H), 7.20 (t, *J* = 7.2 Hz, 2H), 7.07 (dd, *J* = 6.4, 2.8 Hz, 1H), 3.83 (s, 3H), 1.82 (d, *J* = 3.1 Hz, 2H), 1.65 (s, 4H), 1.45 - 1.33 (m, 1H), 1.26 - 1.14 (m, 5H), 1.03 (s, 2H), 0.77 (s, 6H). |
| LSX526a | 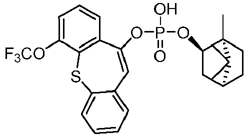 | ¹H NMR (400 MHz, DMSO-*d*₆) δ 7.77 - 7.55(m, 1H), 7.46 (d, *J* = 5.0 Hz, 2H), 7.44 - 7.37 (m, 2H), 7.36 - 7.30 (m, 1H), 7.29 - 7.20 (m, 2H), 3.83 (dd, *J* = 9.6, 1.6 Hz, 1H), 1.79 - 1.67 (m, 1H), 1.67 - 1.54 (m, 2H), 1.48 - 1.40 (m, 1H), 1.39 - 1.27 (m, 1H), 1.07 - 0.94 (m, 7H), 0.93 - 0.81 (m, 4H). |
| LSX526j | 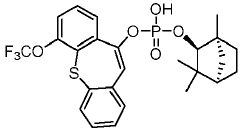 | ¹H NMR (400 MHz, DMSO-*d*₆) δ 7.75 (p, *J* = 4.2 Hz, 1H), 7.48 (d, *J* = 4.8 Hz, 2H), 7.46 - 7.38 (m, 2H), 7.36 - 7.29 (m, 1H), 7.29 - 7.20 (m, 2H), 4.60 (t, *J* = 8.8 Hz, 1H), 2.15 -1.90 (m, 2H), 1.68 - 1.56 (m, 1H), 1.54 - 1.48 (m, 1H), 1.13 - 1.09 (m, 3H), 0.88 - 0.70 (m, 9H). |
| LSX527 | 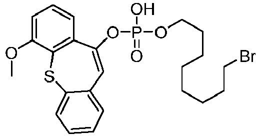 | ¹H NMR (400 MHz, DMSO-*d*₆) δ 7.45 (d, *J* = 7.2 Hz, 1H), 7.37 -7.13 (m, 6H), 7.06 (d, *J* = 7.2 Hz, 1H), 3.83 (s, 3H), 3.79 - 3.70 (m, 2H), 3.51 - 3.40 (m, 2H), 1.77 - 1.61 (m, 2H), 1.54 - 1.38 (m, 2H), 1.34 - 1.09 (m, 8H). |
| LSX548 | 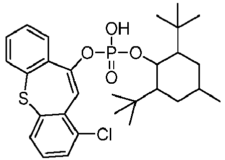 | ¹H NMR (400 MHz, DMSO-*d*₆) δ 7.62 - 7.57 (m, 1H), 7.57 - 7.43 (m, 5H), 7.35 (t, *J* = 7.9 Hz, 1H), 7.31 (d, *J* = 1.9 Hz, 1H), 5.21 (d, *J* = 8.3 Hz, 1H), 1.53 - 1.32 (m, 3H), 1.27 - 1.12 (m, 4H), 0.98 (s, 18H), 0.88 (d, *J* = 6.0 Hz, 3H). |
| LSX552 | 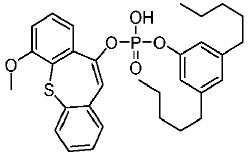 | ¹H NMR (400 MHz, DMSO-*d*₆) δ 7.46 (dd, *J* = 7.6, 1.6 Hz, 1H), 7.37 - 7.15 (m, 5H), 7.07 (dd, *J* = 10.8, 7.6 Hz, 2H), 6.84 (s, 2H), 6.62 (s, 1H), 2.41 (t, *J* = 7.6 Hz, 4H), 1.49 - 1.37 (m, 4H), 1.23 - 1.13 (m, 8H), 0.80 (t, *J* = 6.8 Hz, 6H). |
| LSX602 | 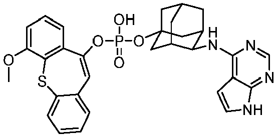 | ^{I}HNMR (400 MHz, DMSO-*d*₆) δ 11.51 (s, 1H), 8.07 (s, 1H), 7.45 (dd, *J* = 7.7, 1.3 Hz, 1H), 7.34 - 7.25 (m, 4H), 7.25 - 7.16 (m, 2H), 7.07 (dd, *J* = 6.0, 3.4 Hz, 1H), 7.03 (dd, *J* = 3.4, 2.3 Hz, 1H), 6.86 - 6.69 (m, 2H), 4.26 (d, *J* = 6.5 Hz, 1H), 3.83 (s, 3H), 2.23 - 2.14 (m, 4H), 2.14 - 1.96 (m, 7H), 1.36 (d, *J* = 12.2 Hz, 2H). |
| LSX644 | 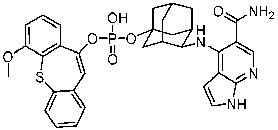 | ¹H NMR (400 MHz, DMSO-*d*₆) δ 11.66 (s, 1H), 10.16 (s, 1H), 8.39 (s, 1H), 7.86 (s, 1H), 7.47 (d, *J* = 7.6 Hz, 1H), 7.33 - 7.28 (m, 3H), 7.26 - 7.18 (m, 2H), 7.13 - 7.06 (m, 2H), 6.34 (dd, *J* = 3.7, 1.9 Hz, 1H), 4.13 (d, *J* = 8.0 Hz, 1H), 3.84 (s, 3H), 2.26 (d, *J* = 11.4 Hz, 2H), 2.19 - 2.00 (m, 7H), 1.82 (d, *J* = 12.8 Hz, 2H), 1.43 (d, *J* = 12.6 Hz, 2H). |
| LSX694 | 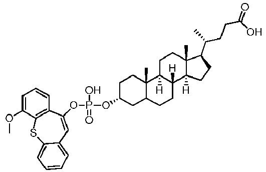 | ¹H NMR (400 MHz, DMSO-*d*₆) δ 7.53 - 7.40 (m, 1H), 7.39 - 7.14 (m, 4H), 7.14 - 7.01 (m, 1H), 4.13 - 4.01 (m, 1H), 3.82 (s, 3H), 2.28 - 2.03 (m, 4H), 1.99 - 0.69 (m, 31 H), 0.59 (s, 3H). |
| LSX710 | 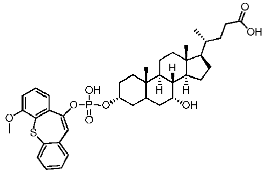 | ¹H NMR (400 MHz, DMSO-*d*₆) δ 7.44 (d, *J* = 7.6 Hz, 1H), 7.38 - 7.12 (m, 6H), 7.06 (d, *J* = 7.6 Hz, 1H), 4.15 - 4.04 (m, 1H), 3.83 (s, 3H), 3.67 - 3.50 (m, 1H), 2.36 - 2.17 (m, 2H), 2.17 - 2.02 (m, 1H), 1.84 - 1.45 (m, 9H), 1.46 - 0.66 (m, 25H), 0.58 (s, 3H). |
| LSX726 | 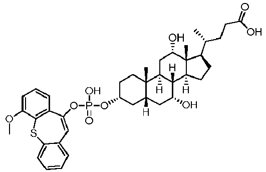 | ¹H NMR (400 MHz, Methanol-*d*₄) δ 7.55 - 7.45 (m, 1H), 7.34 - 7.22 (m, 4H), 7.19 (d, *J* = 2.4 Hz, 1H), 7.08 (dd, *J* = 7.6, 1.6 Hz, 1H), 4.20 - 4.10 (m, 1H), 3.96 - 3.91 (m, 1H), 3.88 (s, 3H), 3.81 - 3.74 (m, 1H), 2.54 - 2.13 (m, 6H), 2.03 - 1.78 (m, 7H), 1.78 - 1.71 (m, 4H), 1.67 - 1.38 (m, 10H), 131-126 (m, 2H), 1.11 - 1.00 (m, 3H), 1.00 - 0.82 (m, 9H), 0.70 (s, 3H). |

### Example 2

### Synthesis of Compound LSX442

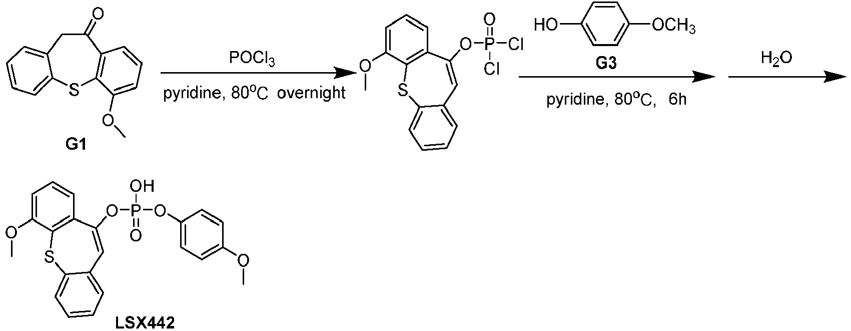

The raw material G1 (200 mg, 0.78 mmol) was dissolved in anhydrous pyridine (5 mL), redistilled POCl₃ (0.078 mL, 0.86 mmol) was added dropwise under the protection of argon, and reacted at 80°C overnight. The mixture was cooled to room temperature on the next day, and G3 (290 mg, 2.34 mmol) was added dropwise to the system. Then the system was heated up to 80°C and reacted for 6h. The mixture was then cooled to room temperature, quenched with water, adjusted to pH7 with 1 mol/L hydrochloric acid and extracted with ethyl acetate. After the aqueous phase was back-extracted three times, the mixture was washed with saturated brine, dried over anhydrous Na₂SO₄, filtered, concentrated, and subjected to silica gel column chromatography (DCM:MeOH = 15:1-10:1) and dried by rotary evaporation to obtain the target compound LSX442 (40 mg, 12%, white solid). ¹H NMR (400 MHz, DMSO-*d*₆) δ 7.46 (d, *J* = 7.2 Hz, 1H), 7.29-7.04 (m, 9H), 6.78 (d, *J=* 8.4 Hz, 2H), 3.83(s, 3H), 3.67(s, 3H).

The following compounds were synthesized by the same method.

| No. | Structure formula | ¹H NMR data |
|---|---|---|
| LSX431 | 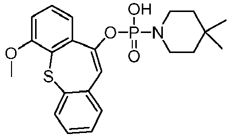 | ¹H NMR (400 MHz, DMSO-*d*₆) δ 7.52 - 7.43 (m, 1H), 7.41 - 7.12 (m, 6H), 7.12 - 7.03 (m, 1H), 3.83 (s, 3H), 3.24 - 2.91 (m, 4H), 1.35 - 1.00 (m, 4H), 0.75 (s, 6H). |
| LSX440 | 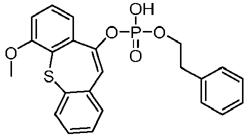 | ¹H NMR (400 MHz, DMSO-*d*₆) δ 7.44 (d, *J* = 7.6 Hz, 1H), 7.29 - 7.13 (m, 1 1H), 7.06 (d, *J* = 7.6 Hz, 1H), 3.92 (t, *J* = 7.2 Hz, 2H), 3.83 (s, 3H), 2.80 (t, *J* = 7.2 Hz, 2H). |
| LSX447 | 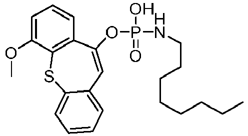 | ¹H NMR (400 MHz, DMSO-*d*₆) δ 7.43 (d, *J* = 7.6 Hz, 1H), 7.36 - 7.10 (m, 6H), 7.03 (d, *J* = 7.6 Hz, 1H), 3.82 (s, 3H), 2.96 - 2.67 (m, 2H), 1.42 - 1.26 (m, 2H), 1.23 - 1.01 (m, 10H), 0.80 (t, *J* = 6.8 Hz, 3H). |
| LSX504 | 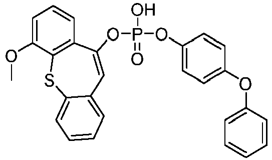 | ¹H NMR (400 MHz, DMSO-*d*₆) δ 7.45 (d, *J=* 7.2 Hz, 1H), 7.39 - 7.01 (m, 12H), 6.90 (d, *J* = 8.0 Hz, 4H), 3.82 (s, 3H). |

### Example 3

### Preparation of compound LSX432

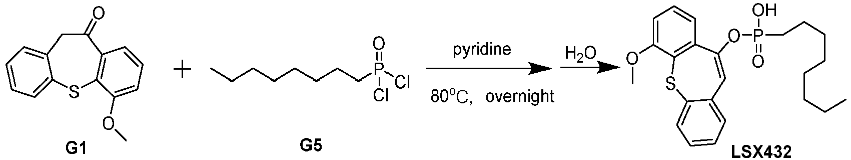

The raw material G1 (200 mg, 0.78 mmol) was dissolved in anhydrous pyridine (5 mL), and G5 (0.480 mL, 2.34 mmol) was added dropwise under the protection of argon, and reacted overnight at 80°C. The mixture was cooled to room temperature, quenched with water, adjusted to pH7 with 1 mol/L hydrochloric acid and extracted with ethyl acetate. After the aqueous phase was back-extracted three times, the mixture was washed with saturated brine, dried over anhydrous Na₂SO₄, filtered, concentrated, and subjected to silica gel column chromatography (DCM:MeOH = 15:1-10:1) and dried by rotary evaporation to obtain the target compound LSX432 (120 mg, 35%, white solid). ¹H NMR (400 MHz, DMSO-*d*₆) δ 7.43 (d, *J* = 7.2 Hz, 1H), 7.39 - 7.10 (m, 6H), 7.05 (d, *J=* 7.2 Hz, 1H), 3.83 (s, 3H), 1.49 - 1.35 (m, 4H), 1.30 - 1.03 (m, 10H), 0.89 - 0.79 (m, 3H).

### Example 4

### Preparation of compound LSX419

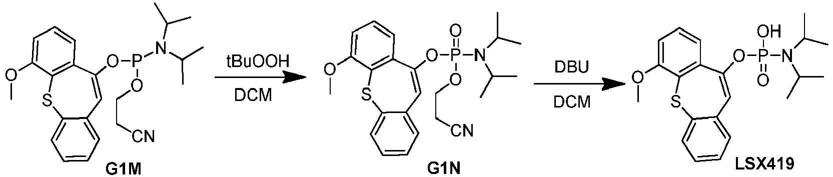

The intermediate G1M (368 mg, 0.78 mmol) was dissolved in anhydrous dichloromethane, and tert-butyl hydroperoxide (70% in water) (0.094 ml, 0.63 mmol) was added and reacted for 1h. The mixture was neutralized with saturated sodium sulfite solution, and extracted with ethyl acetate. After the aqueous phase was back-extracted three times, the mixture was washed with saturated brine, dried over anhydrous Na₂SO₄, filtered, concentrated, and subjected to silica gel column chromatography (PE:AcOEt = 10:1-2:1) to obtain intermediate G1N. G1N was dissolved in anhydrous dichloromethane and 1,8-diazabicycloundec-7-ene (0.094 ml, 0.63 mmol) was added and reacted for 30 minutes, adjusted to pH 7 with 1 mol/L HCl and extracted with ethyl acetate. After the aqueous phase was back-extracted three times, the mixture was washed with saturated brine, dried over anhydrous Na₂SO₄, filtered, concentrated, and subjected to silica gel column chromatography (DCM:MeOH = 15:1-10:1) and dried by rotary evaporation to obtain the target compound LSX419 (120 mg, 18%, white solid). ¹H NMR (400 MHz, DMSO-*d*₆) δ 7.49 (d, *J=* 7.2 Hz, 1H), 7.38-7.24 (m, 5H), 7.20 (dd, *J* = 7.2, 1.2 Hz, 1H), 7.13 (dd, *J* = 7.2, 1.2 Hz, 1H), 3.85 (s, 3H), 3.44 (dp, *J* = 20.0, 6.8 Hz, 2H), 1.15 (d, *J* = 6.8 Hz, 12H).

### Example 5

### Preparation of compound LSX784

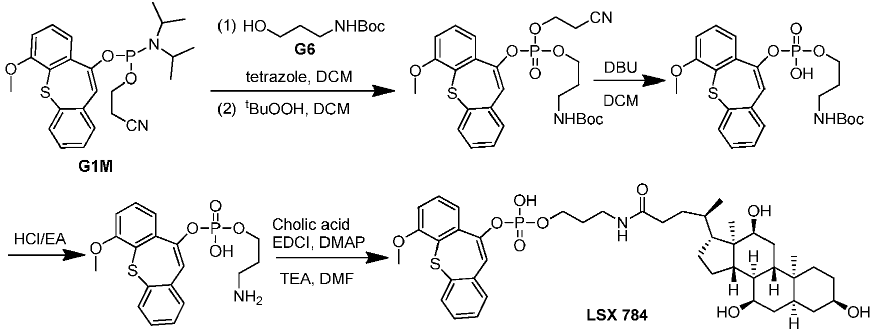

The intermediate G1M (920 mg, 0.78 mmol) was dissolved in anhydrous dichloromethane, and tetrazole (107 mg, 1.575 mmol) and G6 (0.242 ml, 1.575 mmol) were added and reacted for 4h. Then tert-butyl hydroperoxide (70% in water) (0.235 ml, 1.575 mmol) was added and reacted for 1h. The mixture was neutralized with saturated sodium sulfite solution, and extracted with ethyl acetate. After the aqueous phase was back-extracted three times, the mixture was washed with saturated brine, dried over anhydrous Na₂SO₄, filtered, concentrated, and subjected to silica gel column chromatography (PE:AcOEt = 10:1-2:1) to obtain an intermediate. The intermediate was dissolved in anhydrous dichloromethane, and 1,8-diazabicycloundec-7-ene (0.235 ml, 1.575 mmol) was added and reacted for 30 mins. The mixture was adjusted to pH 7 with 1 mol/L HCl, and extracted with ethyl acetate. After the aqueous phase was back-extracted three times, the mixture was washed with saturated brine, dried over anhydrous Na₂SO₄, filtered, concentrated, and subjected to silica gel column chromatography (DCM:MeOH = 15:1-10:1) and dried by rotary evaporation to obtain an intermediate. The intermediate was dissolved in ethyl acetate hydrochloride solution and reacted for 2 h at room temperature and then the solvent was removed at low temperature under reduced pressure. The residue was dried under vacuum and cholic acid (122 mg, 0.298 mmol), EDCI (86 mg, 0.448 mmol) and DMAP (109 mg, 0.892 mmol) were added, N,N-dimethylformamide and triethylamine were used as solvents, and the reaction was carried out at room temperature for 2 d. The solvents were removed under reduced pressure, and the residue was concentrated and subjected to a silica gel column chromatography (DCM:MeOH:AcOH = 10:1:0.1- 5:1:0.1) to obtain LSX784 (16 mg, 1.05%, white solid). ¹H NMR (400 MHz, DMSO-*d*₆) δ 7.46 (d, *J* = 7.6 Hz, 1H), 7.38 - 7.17 (m, 6H), 7.13 - 7.06 (m, 1H), 4.31 (d, *J* = 4.4 Hz, 1H), 4.16 - 4.06 (m, 1H), 4.00 (d, *J=* 3.2 Hz, 1H), 3.94 - 3.80 (m, 5H), 3.80 - 3.74 (m, 1H), 3.67 - 3.55 (m, 1H), 3.09 (q, *J=* 6.8 Hz, 2H), 2.28 - 2.06 (m, 2H), 2.07 - 1.91 (m, 1H), 1.86 - 1.70 (m, 4H), 1.72 - 1.57 (m, 4H), 1.49 - 1.09 (m, 15H), 1.04 - 0.77 (m, 12H), 0.59 (s, 3H).

### Example 6

### (1) Purpose of the experiment

GPR84 antagonistic activity of the compounds of the invention was determined.

### (2) Material source

The human GPR84 cell line was obtained by transfecting a HEK293 cell line with a plasmid encoding GPR84 and Gα16 proteins. Fluorescent dye Fluo-4AM was purchased from Invitrogen.

### (3) Test principle

Intracellular Ca²⁺ ion is a very important second messenger of G protein-coupled receptor signaling pathway. When combined with Gα16 protein-coupled GPR84 and agonist, intracellular Ca²⁺ ion concentration can be significantly increased. Fluo-4 is a Ca²⁺ ion-specific fluorescent probe that binds quantitatively to Ca²⁺ ions and emits fluorescence. Therefore, the agonistic or antagonistic activity of the compound can be detected in a 96-well or 384-well flat-bottomed microplate using a fluorescence assay.

Detection of receptor inhibitory effect by GPR84 antagonist: After incubated with fluorescent dye Fluo-4, GPR84 cells were incubated with different concentrations of antagonistic compounds for a period of time to occupy the binding site of agonist to GPR84 (antagonistic binding site). A certain concentration of agonist (6-n-octylaminouracil, 6-OAU) was added to compete with the antagonist compound for binding sites. A light source with a wavelength of 485 nm was used for excitation and changes in the fluorescence intensity of the dye caused by changes in intracellular calcium concentration at 525 nm was detected. The half-inhibitory concentration (IC₅₀) of the compound was calculated by using GraphPad PRISM software.

### (4) Experimental process

Preparation of HBSS: 0.4 g/L KCl (5.4 mM), 0.12 g/L Na₂HPO₄·12H₂O (0.3 mM), 0.06 g/L KH₂PO₄ (0.4 mM), 0.35 g/L NaHCO₃ (4.2 mM), 0.14 g/L CaCl₂ (1.3 mM), 0.10 g/L MgCl₂·6H₂O (0.5 mM), 0.05 g/L MgSO₄ (0.6 mM), 8.0 g/L NaCl (137 mM). The above ingredients were weighed and dissolved in ultrapure water. pH was adjusted to 7.4 by using hydrochloric acid or NaOH solution. The mixture was filtered and stored at 4 °C for one month.

Preparation of calcium buffer: 560 mM D-glucose (100X) aqueous stock solution and 250 mM 1,2-diphenyl-4-(2-phenylsulfinyl)ethyl-3,5-pyrazolidinedione (1000X) stock solution were firstly prepared. Then BSA (0.5g), 560 mM D-glucose stock solution (1 mL) and 250 mM 1,2-diphenyl-4-(2-phenylsulfinyl)ethyl-3,5-pyrazolidinedione stock solution (100 µL) were added to 100 mL HBSS to a final concentration of 0.5% BSA, 5.6 mM D-glucose, 250 µM 1,2-diphenyl-4-(2-phenylsulfinyl)ethyl-3,5-pyrazolidinedione and mixed. The buffer was prepared on site.

Preparation of dye: 2 mM Fluo-4 AM (1000X, purchased from Invitrogen) in DMSO (1 µL) and 3% Cremophor EL (100X, purchased from SigmaAldrich) in PBS (10 µL) were mixed, and then 1 mL of calcium buffer was added and mixed well.

The cells were seeded in a 96-well plate at a density of 4×10⁴ cells/well. After 24 hours of culture, the culture medium was removed, and 40 µL of Fluo-4AM fluorescent staining solution was added to each well and incubated in a 37°C incubator for 40 minutes. After the dye was aspirated and discarded, 50 µL of the tested compound was added and incubated at room temperature for 10 minutes. 25 µL of 6-OAU (the effective concentration after dilution is around the EC80 of the agonist) was added to stimulate on the Flex Station III microplate reader, and real-time change value of Fluo-4AM dye fluorescence intensity caused by the change of intracellular calcium ion flow (excitation wavelength 485nm, detection wavelength 525nm).

### (5) Experimental results

**Table 1. IC₅₀ and LogP of the compounds**

| **Compound** | **IC₅₀ *^{a}*** | **CLogP *^{b}*** | **ALogP *^{c}*** | **Compound** | **IC₅₀** | **CLogP** | **ALogP** |
|---|---|---|---|---|---|---|---|
| XYF573c | *** | 6.419 | 6.8 | LSX470b | ** | 5.346 | 5.076 |
| LSX350 | * | 1.647 | 2.818 | LSX472a | *** | 5.51 | 5.152 |
| LSX406 | * | 2.095 | 2.6 | LSX472b | *** | 5.51 | 5.152 |
| LSX406-2 | * | 3.763 | 4.6 | LSX472j | *** | 5.51 | 5.287 |
| LSX412 | * | 3.421 | 4.4 | LSX474-2 | ** | 5.524 | 5.843 |
| LSX419 | ** | 5.476 | 3.83 | LSX474-3 | ** | 5.754 | 5.863 |
| LSX420 | * | 1.279 | 2.66 | LSX474a | *** | 5.644 | 5.956 |
| LSX420-3 | ** | 4.292 | 5.06 | LSX474b | *** | 5.644 | 5.956 |
| LSX426j | * | 5.317 | 4.75 | LSX476a | *** | 6.326 | 5.833 |
| LSX430 | * | 3.564 | 3.601 | LSX476b | *** | 6.326 | 5.833 |
| LSX430-2 | ** | 3.953 | 4.42 | LSX472c | *** | 5.51 | 5.152 |
| LSX431 | * | 6.725 | 3.752 | LSX472d | *** | 5.51 | 5.152 |
| LSX432 | ** | 5.715 | 5.632 | LSX472e | *** | 5.51 | 5.287 |
| LSX432-2a | * | 4.197 | 4.996 | LSX472f | *** | 5.51 | 5.287 |
| LSX432-2b | * | 4.197 | 4.929 | LSX472g | *** | 5.51 | 5.152 |
| LSX432-2c | * | 4.197 | 4.929 | LSX472h | ** | 5.51 | 5.152 |
| LSX432-3 | * | 4.237 | 5.133 | LSX476c | ** | 6.326 | 5.833 |
| LSX434 | ** | 4.821 | 5.516 | LSX476d | ** | 6.326 | 5.833 |
| LSX434-2 | * | 3.133 | 3.18 | LSX476e | ** | 6.326 | 5.968 |
| LSX440 | * | 3.744 | 4.723 | LSX476f | ** | 6.326 | 5.968 |
| LSX442 | ** | 3.34 | 4.38 | LSX476g | *** | 6.326 | 5.833 |
| LSX442a | ** | 5.557 | 5.17 | LSX476h | ** | 6.326 | 5.833 |
| LSX442j | * | 5.557 | 5.3 | LSX476j | ** | 6.326 | 5.968 |
| LSX444 | ** | 4.392 | 5.785 | LSX480 | ** | 4.847 | 5.724 |
| LSX444-2 | ** | 4.572 | 4.683 | LSX482a | ** | 6.036 | 6.706 |
| LSX446 | ** | 4.866 | 5.527 | LSX482b | *** | 6.036 | 6.706 |
| LSX446-2 | * | 4.716 | 5.315 | LSX482c | ** | 6.036 | 6.706 |
| LSX447 | ** | 7.257 | 5.235 | LSX491a | ** | 4.284 | 5.144 |
| LSX448 | ** | 5.35 | 5.972 | LSX492 | * | 3.78 | 4.92 |
| LSX452 | ** | 6.166 | 6.653 | LSX496 | * | 6.275 | 6.783 |
| LSX454 | * | 4.848 | 5.589 | LSX498 | ** | 5.344 | 5.374 |
| LSX456j | * | 5.236 | 4.73 | LSX504 | * | 5.519 | 5.96 |
| LSX457 | * | 3.164 | 4.289 | LSX526a | ** | 6.619 | 7.29 |
| LSX460 | ** | 5.125 | 5.704 | LSX526j | *** | 6.619 | 7.42 |
| LSX460a | ** | 5.756 | 5.37 | LSX527 | ** | 5.203 | 6.905 |
| LSX460j | ** | 5.756 | 5.51 | LSX548 | * | 8.705 | 8.080 |
| LSX462-3 | ** | 5.879 | 6.428 | LSX552 | ** | 8.651 | 9.017 |
| LSX462a | ** | 4.595 | 5.303 | LSX602 | *** | 4.201 | 4.5 |
| LSX462b | ** | 4.595 | 5.3 | LSX644 | * | 4.231 | 3.56 |
| LSX466 | * | 4.623 | 5.686 | LSX694 | ** | - | 7.657 |
| LSX468a | ** | 5.347 | 6.075 | LSX710 | ** | - | 6.488 |
| LSX468b | ** | 5.347 | 6.075 | LSX726 | * | - | 5.386 |
| LSX470a | ** | 4.306 | 4.961 | LSX784 | * | - | 5.191 |

| | | | | | | | |
|---|---|---|---|---|---|---|---|
| *^{a}* IC₅₀: * 1 - 10 µM; ** 0.1 -1 µM; *** <0.1 µM *^{b}* CLogP was calculated by ChemBioDraw software (Ultra 12.0) of CambridgeSoft Company, USA. The structures used in the calculations were all the free acid forms of the compounds. *^{c}* ALogP was calculated by Maestro software (version 11.2) of Schrödinger Company, USA. All compounds were calculated based on the ionization state at pH=7. And the data of ALogP and CLogP of compound LSX431 were quite different due to the protonation of nitrogen atom of phosphoramide. | | | | | | | |

### (6) Analysis of results

The results in Table 1 show that the IC₅₀ values of the compounds of the present invention are all less than 10 µM, the IC₅₀ values of some compounds are 0.1-1 µM, and the IC₅₀ values of some compounds are even less than 100 nM, indicating that the compounds of the present invention can be used as GPR84 antagonists. In addition, compared with the existing compound XYF573c (IC₅₀=26.2nM, CLogP=6.419, ALogP=6.80), 30 compounds have a CLogP between 5 and 6, 37 compounds have a CLogP less than 5, 47 compounds hava an ALogP between 5 and 6, and 26 compounds have an ALogP less than 5, which are significantly lower than the CLogP and ALogP of XYF573c.

### Example 7

### (1) Purpose of the experiment

The tissue distribution experiment of compounds XYF573c and LSX472a of the present invention in ICR mice was carried out, and their distribution in plasma and organs and oral exposure (AUC₀₋₈ₕ) were compared.

### (2) Source of material

ICR mice were purchased from Shanghai Experimental Animal Research Center.

### (3) Test principle

The concentrations of the compounds in plasma and various organs of ICR mice were detected by LC-MS.

### (4) Experimental process

During the experiment, the mice were fasted for more than 12 hours, had free access to water, and provided with food 2 hours after administration.

Both compounds XYF573c and LSX472a were administered orally, the dose was 5 mg/kg, and the solvent was DMSO/0.5% HPMC (5/95, v/v), N=3.

At 0.5h, 2h and 8h after oral administration, about 20 µL of mouse heart blood, portal vein plasma or 20 mg of liver, lung, and ileum tissues were taken in clean centrifuge tubes, 200 µL of MeOH/ACN (50/50, v/v) was added and spun in a vortex mixer for 1 min. The mixtures were centrifuged at 15,000 rpm for 5 min and 20 µL of supernatants were mixed with 20 µL of ACN/water (1/1, v/v) and quantified by LC-MS.

The values of AUC₀₋₈ₕ were calculated by trapezoidal method based on the concentrations of the compound at 0.5h, 2h and 8h.

### (5) Experimental results

A reduction in cLogP is essential to improve the oral absorption of the compound. The cLogP of the compound LSX472a of the present invention is 5.51, which is 0.9 lower than that of XYF573c. This change is also demonstrated by a 13-fold increase in oral exposure (Plasma AUC₀₋₈ₕ) of LSX472a compared to XYF573c (Figure 1 and Table 1). Moreover, exposure of LSX472a in the intestinal tract (such as Ileum) was significantly increased compared to XYF573c. Therefore, the structure expansion of the GPR84 antagonists in the present invention improves the drug-like properties of these compounds, increases the oral absorption, and helps to improve the target tissue distribution of the compounds.

**Table 1 Oral exposure of compounds XYF573c and LSX472a**

| plasma or tissue | AUC₀₋₈ₕ (h*ng/mL for plasma or h*ng/g for tissues) | |
|---|---|---|
| | XYF573c | LSX472a |
| Heart plasma | 445.09 | 7751.91 |
| Portal vein plasma | 666.53 | 8815.83 |
| Lung | 1159.74 | 745.34 |
| Liver | 11105.83 | 4387.83 |
| Ileum | 24447.42 | 62902.60 |

All documents mentioned in the present application are incorporated herein by reference, just as each document is cited separately as a reference. In addition, it should be understood that various modifications and changes may be made by those skilled in the art after reading the above teachings of the present invention. These equivalent forms are also within the scope defined by the claims appended hereto.

## Claims

1. A compound represented by general formula (I), or an enantiomer, diastereoisomer, racemate, or a pharmaceutically acceptable salt thereof, 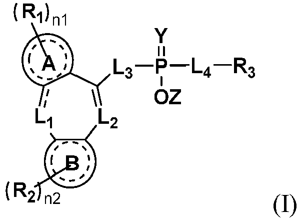
wherein Y is O or S;
Z is H, or an ion of the following metals: Li, Na, K, Ca, Mg, Cu, Fe, Zn, Al, Mn, or a conjugate acid of the following bases: NH₃, arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxycobalamin, isopropylamine, lysine, methyl glucosamine, morpholine, piperazine, piperidine, polyamine resin, procaine, purine, theobromine, triethylamine, trimethylamine, tripropylamine, trometamol, imidazole;
L₁ is none, O, S, -CH=CH-, -CO-, -C(=CH₂)-, substituted or unsubstituted C₁-C₆ alkylene, -NH-, -N(C₁-C₄ alkyl)-, C₃-C₆ cycloalkyl or C₃-C₆ heterocycloalkyl, and the substitution means that there is one or more substituents selected from the group consisting of C₁-C₆ alkyl, C₁-C₆ alkoxy, halogen, and hydroxyl;
L₂ is none, CH;
ring A and ring B are each independently a C₆-C₁₀ aromatic ring, a C₃-C₁₀ cycloalkane ring, a C₃-C₁₀ heterocycloalkane ring, or a C₃-C₁₀ heteroaryl ring;
n1 and n2 are each independently 0, 1, 2, 3 or 4; R₁ and R₂ are each independently -OH, -SH, -NH₂, F, Cl, Br, I, -CᵣH₂ᵣ-L₇-CₛH₂ₛ₊₁, -CᵣH₂ᵣ-N(CₜH₂ₜ₊₁)-CₛH₂ₛ₊₁, substituted or unsubstituted C₁-C₆ alkyl, the above substitution means that there is one or more substituents selected from the group consisting of halogen, hydroxyl, amino, -COOC₁-C₆ alkyl, -COOH; each L₇ is independently O, S, NH; each r is independently 0, 1, 2, 3, 4, 5 or 6; each s is independently 0, 1, 2, 3, 4, 5 or 6; and each t is independently 1, 2, 3, 4, 5, or 6;
L₃ and L₄ are each independently O, none, -O(C₁-C₁₀ alkylene)-, -O(C₁-C₁₀ alkylene)NH-, -O(C₁-C₁₀ alkylene)O-, -CONH-, -OCO-, -NH-, -NHCOO-, -N(C₁-C₆ alkyl)-, or C₁-C₁₀ alkylene;
R₃ is H, OH, NH₂, SH, -COOH, substituted or unsubstituted C₁-C₁₀ alkyl, substituted or unsubstituted C₂-C₁₀ alkenyl, substituted or unsubstituted C₂-C₁₀ alkynyl, substituted or unsubstituted 3-12 membered cycloalkane ring, substituted or unsubstituted C₆-C₁₄ aromatic ring, substituted or unsubstituted amino, substituted or unsubstituted 4-10 membered heterocycloalkane ring, substituted or unsubstituted 4-10 membered heteroaromatic ring, cholic acid, lithocholic acid, deoxycholic acid, isolithocholic acid, isodeoxycholic acid, chenodeoxycholic acid, ursodeoxycholic acid, α-muricholic acid, β-muricholic acid, γ-muricholic acid, ω-muricholic acid; and the above substitution means that there is 1, 2, 3, 4, 5 or 6 substituents selected from the following group consisting of amino, C₁-C₁₀ alkyl, halogen, C₆-C₁₀ aromatic ring, C₁-C₆ alkoxy, 3-12 membered cycloalkane ring, -O-CₚH₂ₚ-O-C_{q}H_{2q+1}, nitro, oxo (=O), hydroxyl, carboxyl, -C(O)OC₁ -C₆ alkyl, -O-C₆-C₁₀ aromatic ring; wherein, each p is independently 1, 2, 3, 4, 5 or 6, and each q is independently 1, 2, 3, 4, 5 or 6;
each - - - independently represents a single or double bond.

2. A compound represented by general formula (I), or an enantiomer, diastereoisomer, racemate, or a pharmaceutically acceptable salt thereof, 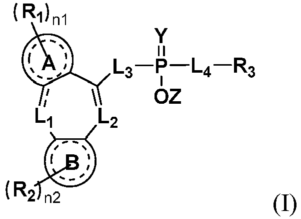
wherein Y is O or S;
Z is H, or an ion of the following metals: Li, Na, K, Ca, Mg, Cu, Fe, Zn, Al, Mn, or a conjugate acid of the following bases: NH₃, arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxycobalamin, isopropylamine, lysine, methyl glucosamine, morpholine, piperazine, piperidine, polyamine resin, procaine, purine, theobromine, triethylamine, trimethylamine, tripropylamine, trometamol, imidazole;
L₁ is none, O, S, -CH=CH-, -CO-, -C(=CH₂)-, substituted or unsubstituted C₁-C₆ alkylene, -NH-, -N(C₁-C₄ alkyl)-, C₃-C₆ cycloalkyl or C₃-C₆ heterocycloalkyl, and the substitution means that there is one or more substituents selected from the group consisting of C₁-C₆ alkyl, C₁-C₆ alkoxy, halogen, and hydroxyl;
L₂ is none, CH;
ring A and ring B are each independently a C₆-C₁₀ aromatic ring, a C₃-C₁₀ cycloalkane ring, a C₃-C₁₀ heterocycloalkane ring, or a C₃-C₁₀ heteroaryl ring;
n1 and n2 are each independently 0, 1, 2, 3 or 4; R₁ and R₂ are each independently -OH, -SH, -NH₂, F, Cl, Br, I, substituted or unsubstituted -CᵣH₂ᵣ-L₇-CₛH₂ₛ₊₁, -CᵣH₂ᵣ-N(CₜH₂ₜ₊₁)-CₛH₂ₛ₊₁, substituted or unsubstituted C₁-C₆ alkyl, the above substitution means that there is one or more substituents selected from the group consisting of halogen, hydroxyl, amino, -COOC₁-C₆ alkyl, -COOH; each L₇ is independently O, S, NH; each r is independently 0, 1, 2, 3, 4, 5 or 6; each s is independently 0, 1, 2, 3, 4, 5 or 6; and each t is independently 1, 2, 3, 4, 5, or 6;
L₃ and L₄ are each independently O, none, -O(C₁-C₁₀ alkylene)-, -O(C₁-C₁₀ alkylene)NH-, -O(C₁-C₁₀ alkylene)O-, -CONH-, -OCO-, -NH-, -NHCOO-, -N(C₁-C₆ alkyl)-, or C₁-C₁₀ alkylene;
R₃ is H, OH, NH₂, SH, -COOH, substituted or unsubstituted C₁-C₁₀ alkyl, substituted or unsubstituted C₂-C₁₀ alkenyl, substituted or unsubstituted C₂-C₁₀ alkynyl, substituted or unsubstituted 3-12 membered cycloalkane ring, substituted or unsubstituted C₆-C₁₄ aromatic ring, substituted or unsubstituted amino, substituted or unsubstituted 4-10 membered heterocycloalkane ring, substituted or unsubstituted 4-10 membered heteroaromatic ring, cholic acid, lithocholic acid, deoxycholic acid, isolithocholic acid, isodeoxycholic acid, chenodeoxycholic acid, ursodeoxycholic acid, α-muricholic acid, β-muricholic acid, γ-muricholic acid, ω-muricholic acid; and the above substitution means that there is 1, 2, 3, 4, 5 or 6 substituents selected from the following group consisting of amino, C₁-C₁₀ alkyl, halogen, C₆-C₁₀ aromatic ring, C₁-C₆ alkoxy, 3-12 membered cycloalkane ring, -O-CₚH₂ₚ-O-C_{q}H_{2q+1}, nitro, oxo (=O), hydroxyl, carboxyl, -C(O)OC₁-C₆ alkyl, -O-C₆-C₁₀ aromatic ring, -NH-(4-10 membered heteroaromatic ring), -NH-(4-10 membered heteroaromatic ring)-CONH₂; wherein each p is independently 1, 2, 3, 4, 5 or 6, and each q is independently 1, 2, 3, 4, 5 or 6;
each - - - independently represents a single or double bond.

3. The compound according to claim 1 or 2, wherein L₁ is independently none, CH₂, O, S, -CO-, -NH-; and
L₂ is independently none, CH.

4. The compound according to claim 1 or 2, wherein R₃ is a substituted or unsubstituted C₁-C₁₀ alkyl, substituted or unsubstituted C₂-C₁₀ alkenyl, substituted or unsubstituted C₂-C₁₀ alkynyl, substituted or unsubstituted 3-12 membered cycloalkane ring, substituted or unsubstituted C₆-C₁₄ aromatic ring, substituted or unsubstituted amino, substituted or unsubstituted 4-10 membered heterocycloalkane ring, substituted or unsubstituted 4-10 membered heteroaromatic ring, cholic acid, lithocholic acid, deoxycholic acid, isolithocholic acid, isodeoxycholic acid, chenodeoxycholic acid, ursodeoxycholic acid, α-muricholic acid, β - muricholic acid, γ-muricholic acid, ω-muricholic acid; and the above substitution means that there is 1, 2, 3, 4, or 5 substituents selected from the following group consisting of amino, C₁-C₁₀ alkyl, halogen, C₆-C₁₀ aromatic ring, C₁-C₆ alkoxy, 3-12 membered cycloalkane ring, -O-CₚH₂ₚ-O-C_{q}H_{2q+1}, nitro, oxo (=O), hydroxyl, carboxyl, -COOC₁-C₆ alkyl, -O-benzene ring, wherein each p is independently 1, 2 or 3, and each q is independently 1, 2 or 3.

5. The compound according to claim 1 or 2, wherein the compound is selected from the group consisting of:

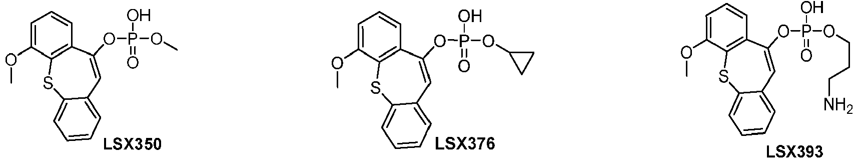

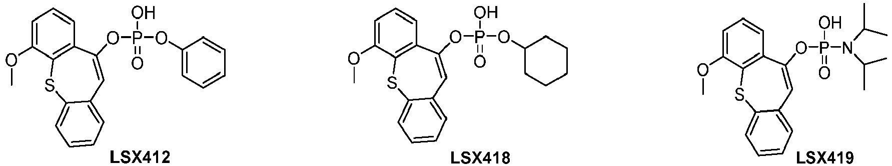

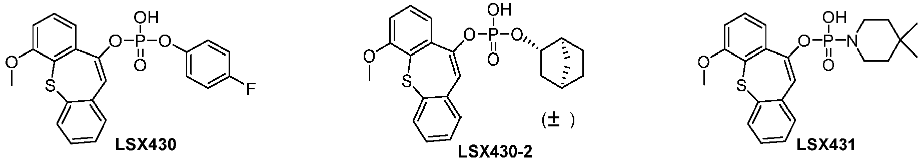

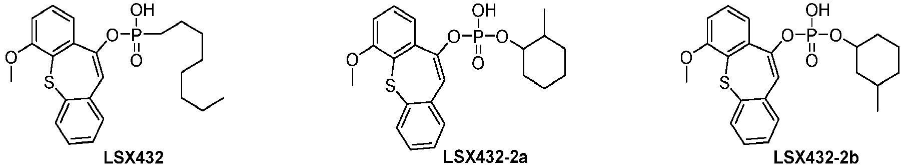

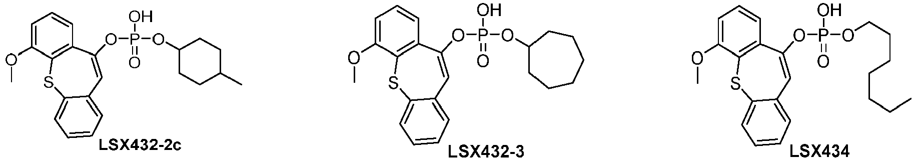

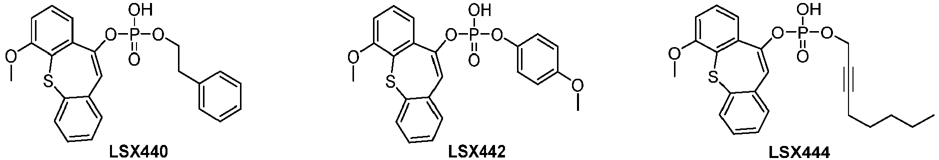

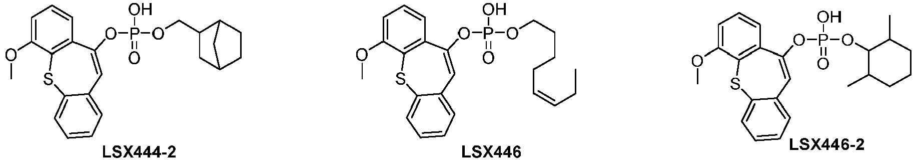

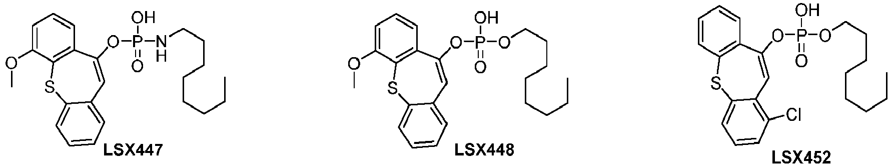

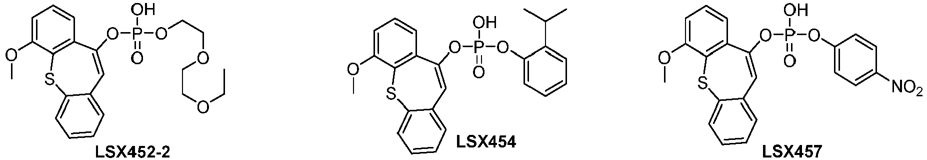

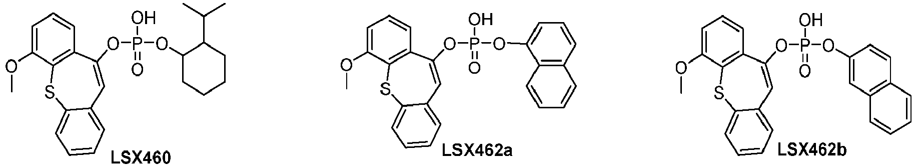

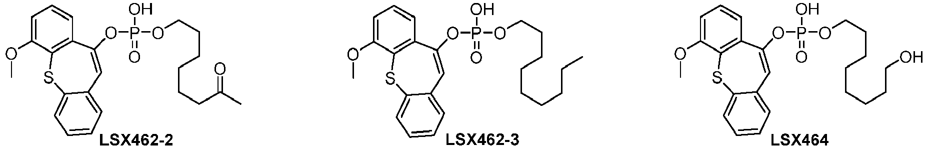

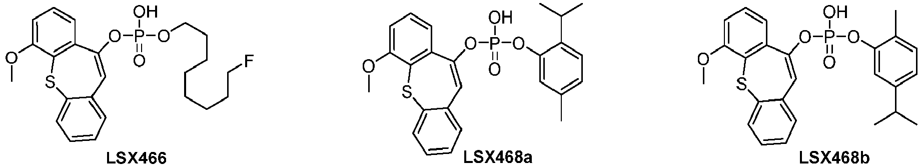

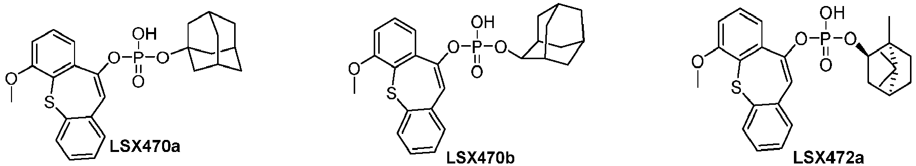

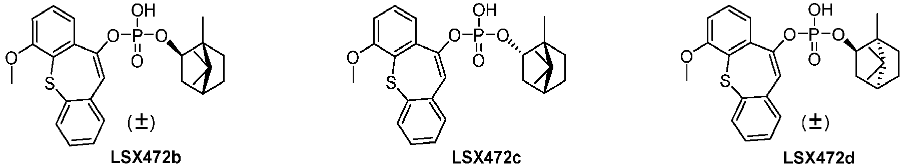

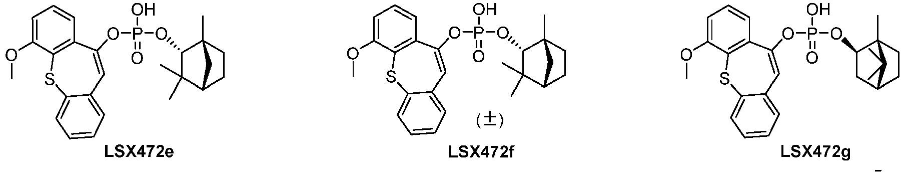

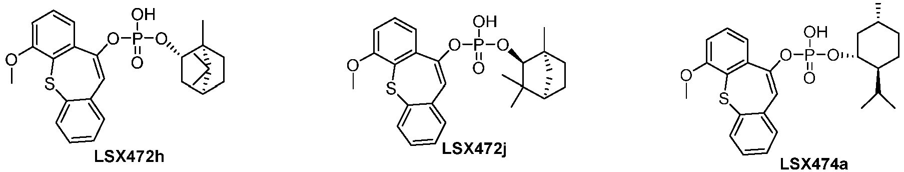

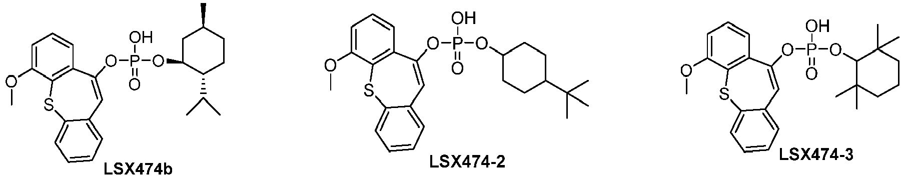

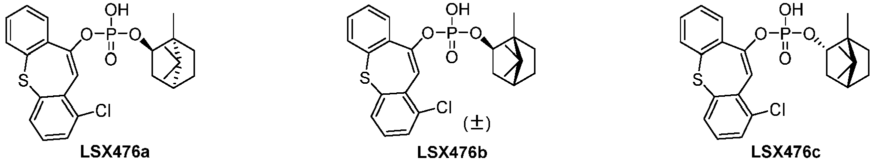

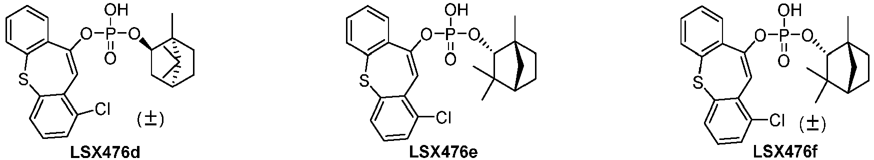

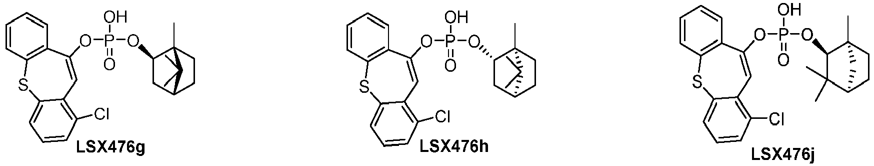

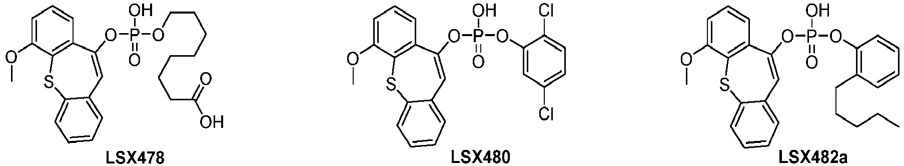

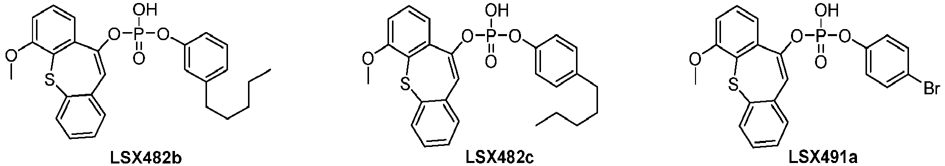

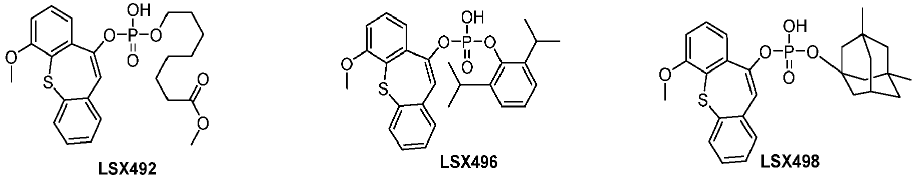

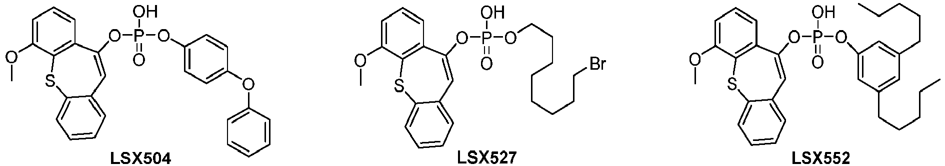

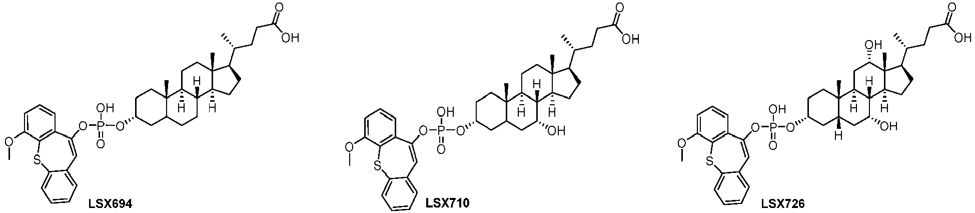

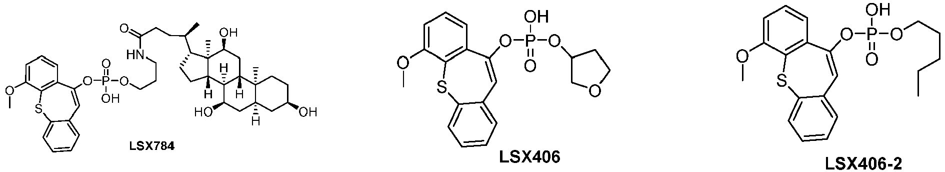

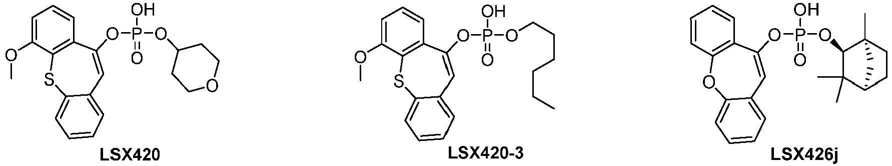

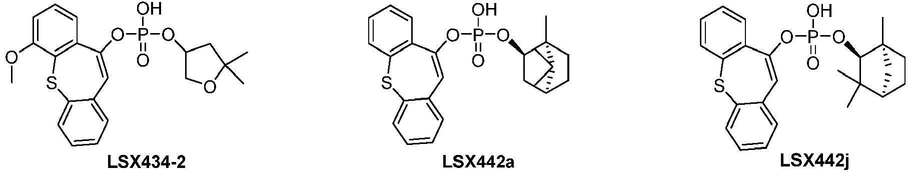

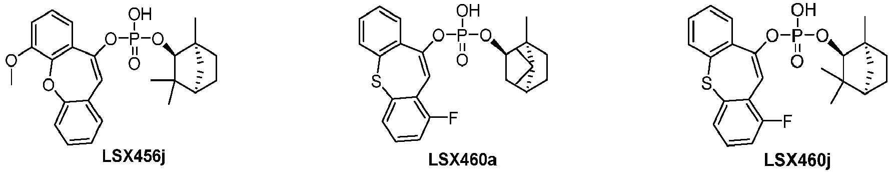

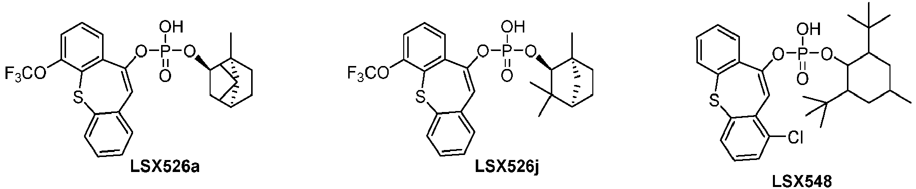

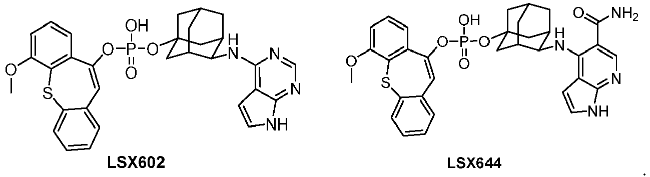

6. A method for preparing the compound of claim 1 or 2, wherein the compound has a structure shown in formula P1, and the method comprises the following step: 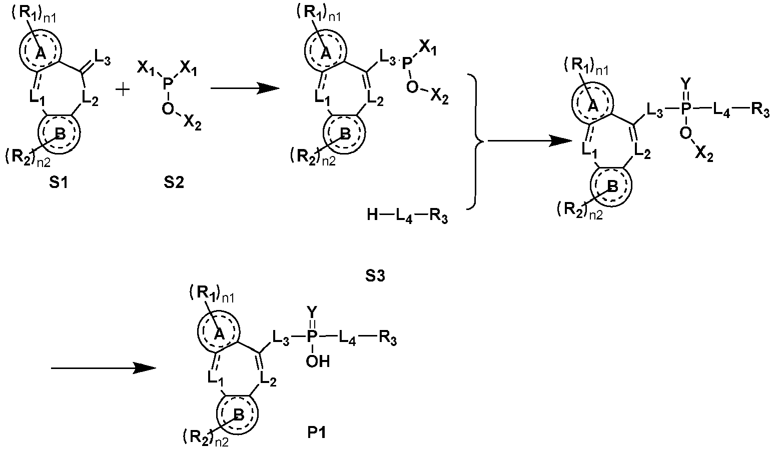
taking a compound of formula S1, a compound of formula S2 and a compound of formula S3 as raw materials and reacting to obtain the compound having the structure shown in formula P1,
wherein R₁, R₂, R₃, L₁, L₂, L₄, L₅, Y, n1, n2, ring A, and ring B are as defined above;
L₃ is O;
each X₁ is independently a dimethylamino, ethylamino, diisopropylamino;
X₂ is a cyanoethyl, allyl, tert-butyl, benzyl.

7. A method for preparing the compound of claim 1 or 2, wherein the compound has a structure shown in formula P1, and the method comprises the following step: 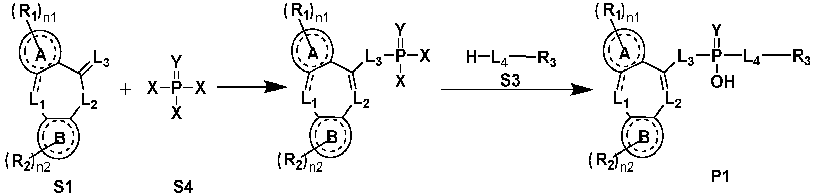
taking a compound of formula S1, a compound of formula S3 and a compound of formula S4 as raw materials and reacting to obtain the compound having the structure shown in formula P1,
wherein R₁, R₂, R₃, L₁, L₂, L₄, L₅, Y, n1, n2, ring A, and ring B are as defined above;
L₃ is O or CH₂; and
each X is independently F, Cl, Br or I.

8. A method for preparing the compound of claim 1 or 2, wherein the compound has a structure shown in formula P1, and the method comprises the following step: 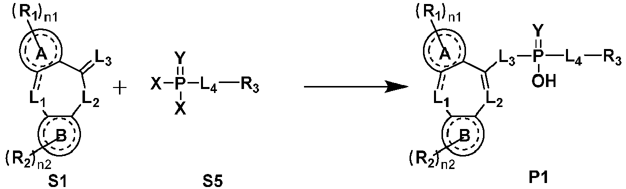
taking a compound of formula S1 and a compound of formula S5 as raw materials and reacting to obtain the compound having the structure shown in formula P1,
wherein R₁, R₂, R₃, L₁, L₂, L₄, L₅, Y, n1, n2, ring A, and ring B are as defined above;
L₃ is O or CH₂; and
each X is independently F, Cl, Br or I.

9. A pharmaceutical composition comprising:
the compound represented by the general formula (I) of any one of claims 1-5, or the enantiomer, diastereoisomer, racemate or pharmaceutically acceptable salt thereof; and
a pharmaceutically acceptable carrier.

10. Use of the compound represented by the general formula (I) of any one of claims 1-5, or the enantiomer, diastereoisomer, racemate or pharmaceutically acceptable salt thereof or the pharmaceutical composition of claim 9:
(i) for the preparation of a GPR84 antagonist;
(ii) as a GPR84 antagonist;
(iii) for the preparation of a medicament for the treatment of a related disease caused by hyper-excitability or high expression of GPR84 receptor.

11. The use of claim 10, wherein the disease is multiple sclerosis, inflammatory bowel disease, fibrosis, neurodegenerative disease or arthritis.
